# EUROPEAN PATENT SPECIFICATION

(11) **EP 1 778 633 B1**
(45) Date of publication and mention of the grant of the patent: **03.12.2008**
(21) Application number: 05751820.1
(22) Date of filing: 17.06.2005
(51) Int. Cl.: C07D 209/42, C07D 401/12, A61K 31/404, A61P 29/00

(54) **INDOLES USEFUL IN THE TREATMENT OF INFLAMMATION**
ZUR BEHANDLUNG VON ENTZÜNDUNGEN GEEIGNETE INDOLE
INDOLES UTILISABLES POUR LE TRAITEMENT D'INFLAMMATIONS

(30) Priority: 18.06.2004 US 580402 P
(43) Date of publication of application: 02.05.2007
(73) Proprietor: Biolipox AB, 171 65 Solna (SE)
(72) Inventor: OLOFSSON, Kristofer, Solna SE-171 65 (SE); SUNA, Edgars Latvian Institute of Organics Synth.,, LV-1006 Riga (LV); PELCMAN, Benjamin, Solna SE-171 65 (SE); OZOLA, Vita Latvian Institute of Organic Synthesis, LV-1006 Riga (LV); KATKEVICS, M.; Latvian Institute of Organic Syn.,, LV-1006 Riga (LV); KALVINS, Ivars Latvian Institute of Organic Syn.,, LV-1006 Riga (LV); SCHAAL, Wesley, 17165 Solna (SE)
(74) Representative: McNeeney, Stephen Phillip
(86) International application number: PCT/GB2005/002396
(87) International publication number: WO 2005/123674

(56) References cited:
- WO-A-00/46195
- WO-A-99/15501

## Description

### Field of the Invention

This invention relates to novel pharmaceutically-useful compounds, which compounds are useful as inhibitors of enzymes belonging to the membrane-associated proteins in the eicosanoid and glutathione metabolism (MAPEG) family. Members of the MAPEG family include the microsomal prostaglandin E synthase-1 (mPGES-1), 5-lipoxygenase-activating protein (FLAP), leukotriene C₄ synthase and microsomal glutathione S-transferases (MGST1, MGST2 and MGST3). The compounds are of potential utility in the treatment of inflammatory diseases including respiratory diseases. The invention also relates to the use of such compounds as medicaments, to pharmaceutical compositions containing them, and to synthetic routes for their production

### Background of the Invention

There are many diseases/disorders that are inflammatory in their nature. One of the major problems associated with existing treatments of inflammatory conditions is a lack of efficacy and/or the prevalence of side effects (real or perceived).

Inflammatory diseases that affect the population include asthma, inflammatory bowel disease, rheumatoid arthritis, osteoarthritis, rhinitis, conjunctivitis and dermatitis.

Inflammation is also a common cause of pain. Inflammatory pain may arise for numerous reasons, such as infection, surgery or other trauma.. Moreover, several diseases including malignancies and cardioavascular diseases are known to have inflammatory components adding to the symptomatology of the patients.

Asthma is a disease of the airways that contains elements of both inflammation and bronchoconstriction. Treatment regimens for asthma are based on the severity of the condition. Mild cases are either untreated or are only treated with inhaled β-agonists which affect the bronchoconstriction clement, whereas patents with more severe asthma typically are treated regularly with inhaled corticosteroids which to a large extent are antiinflammatory in their nature.

Another common disease of the airways with inflammatory and bronchoconstrictive components is chronic obstructive pulmonary disease (COPD). The disease is potentially lethal, and the morbidity and mortality from the condition is considerable. At present, there is no known pharmacological treatment capable of changing the course of the disease.

The cyclooxygenase (COX) enzyme exists in two fonns, one that is constitutively expressed in many cells and tissues (COX-1), and one that is induced by pro-inflammatory stimuli, such as cyokines, during an inflammatory response (COX-2).

COXs metabolise arachidonic acid to the unstable intermediate prostaglandin H₂ (PGH₂). PGH₂ is further metabolised to other prostaglandins including PGE₂, PGF_{2α}, PGD₂, prostacyclin and thromboxane A₂. These arachidonic acid metabolites are known to have pronounced physiological and pathophysiological activity including pro-inflammatory effects.

PGE₂ in particular is known to be a strong pro-inflammatory mediator, and is also known to induce fever and pain. Consequently, numerous drugs have been developed with a view to inhibiting the formation of PGE₂, including "NSAIDs" (non-steroidal anti inflammatory drugs) and "coxibs" (selective COX-2 inhibitors). These drugs act predominantly by inhibition of COX-1 and/or COX-2, thereby reducing the formation of PGE₂.

However, the inhibition of COXs has the disadvantage that it results in the reduction of the formation of all metabolites of arachidonic acid, some of which are known to have beneficial properties. In view of this, drugs which act by inhibition of COXs are therefore known/suspected to cause adverse biological effects. For example, the non-selective inhibition of COXs by NSAIDs may give rise to gastrointestinal side-effects and affect platelet and renal function. Even the selective inhibition of COX-2 by coxibs, whilst reducing such gastrointestinal side-effects, is believed to give rise to cardiovascular problems.

An alternative treatment of inflammatory diseases that does not give rise to the above-mentioned side effects would thus be of real benefit in the clinic. In particular, a drug that inhibits (preferably selectively) the transformation of PGH₂ to the pro-inflammatory mediator PGE₂ might be expected to reduce the inflammatory response in the absence of a corresponding reduction of the formation of other, beneficial arachidonic acid metabolites Such inhibition would accordingly be expected to alleviate the undesirable side-effects mentioned above.

PGH₂ may be transformed to PGE₂ by prostaglandin E synthases (PGES). Two microsomal prostaglandin E synthases (mPGES-1 and mPGES-2), and one cytosolic prostaglandin E syntbase (cPGES) have been described.

The leukotrienes (LTs) are formed from arachidonic acid by a set of enzymes distinct from those in the COX / PGES pathway. Leukotriene B4 is known to be a strong proinflammatory mediator, while the cysteinyl-containing leukotrienes C₄, D₄ and E₄ (CysLTs) are mainly very potent bronchoconstrictors and have thus been implicated in the pathobiology of asthma. The biological activities of the CysLTs are mediated through two receptors designated CysLT₁ and CysLT₂. As an alternative to steroids, leukotriene receptor antagonists (LTRas) have been developed in the treatment of asthma. These drugs may be given orally, but do not control inflammation satisfactorily. The presently used LTRas are highly selective for CysLT₁ It may be hypothesised that better control of asthma, and possibly also COPD, may be attained if the activity of both of the CysLT receptors could be reduced. This may be achieved by developing unselective LTRas, but also by inhibiting the activity of proteins, e.g. enzymes, involved in the synthesis of the CysLTs. Among these proteins, 5-lipoxygenase, 5-lipoxygenase-activating protein (FLAP), and leukotriene C₄ synthase may be mentioned. A FLAP inhibitor would also decrease the formation of the proinflammatory LTB₄.

mPGES-1, FLAP and leukotriene C₄ synthase belong to the membrane-associated proteins in the eicosanoid and glutathione metabolism (MAPEG) family. Other members of this family include the microsomal glutathione S-transferases (MGST1, MGST2 and MGST3). For a review, c.f. P.-J. Jacobsson et al in Am. J. Respir. Crit. Care Med. 161, S20 (2000). It is well known that compounds prepared as antagonists to one of the MAPEGs may also exhibit inhibitory activity towards other family members, *c.f.* J. H Hutchinson et al in J. Med. Chem. 38, 4538 (1995) and D. Claveau et al in J. Immunol. 170, 4738 (2003). The former paper also describes that such compounds may also display notable cross-reactivity with proteins in the arachidonic acid cascade that do not belong to the MAPEG family, e.g. 5-lipoxygenase.

Thus, agents that are capable of inhibiting the action of mPGES-1, and thus reducing the formation of the specific arachidonic acid metabolite PGE₂, are likely to be of benefit in the treatment of inflammation. Further, agents that are capable of inhibiting the action of the proteins involved in the synthesis of the leukotrienes are also likely to be of benefit in the treatment of asthma and COPD.

### Prior Art

Various indole-2-carboxylates, and derivatives thereof, have been disclosed in international patent applications WO 01/30343, WO 96/03377, WO 01/00197 and WO 99/33800, US patents Nos. 5,189,054 and 4,960,786, European patent application EP 483 881 and Italian Patent No. 1303260. However, none of these documents disclose or suggest the use of the indole-2-carboxylates in the treatment of inflammation.

Similar indole-2-carboxylates have been disclosed for potential use in the treatment of inflammation in international patent applications WO 99/07678, WO 99/07351, WO 00/46198, WO 00/46197, WO 00/46195, WO 00/46199, WO 96/18393, WO 02/30895, WO 99/05104, WO 01/32621 and WO 2005/005415, US patents Nos. 5,081,145 and 5,081,138 and European patent applications EP 166 591 and EP 985 666: However, none of these documents disclose such compounds in which an aromatic group is directly attached to the ring system *via*. the indole nitrogen.

International patent application WO 94/13662 and European patent application EP 186 367 also mention indoles for potential use in the treatment of inflammation. However, these documents do not mention or suggest compounds in which the benzenoid moiety of the indole is substituted with an aromatic ring.

International patent applications WO 94/14434, WO 99/43672, WO 98/08818, WO 99/43654 and WO 99/43651 and US patents Nos. 6,500,853 and 6,630,496 also describe structurally similar indoles for such potential use. However, there is no specific disclosure in any of these documents of indole-2-carboxylates in which an aromatic group is directly attached *via* the indole nitrogen.

### Disclosure of the Invention

According to the invention there is provided a compound of formula I, 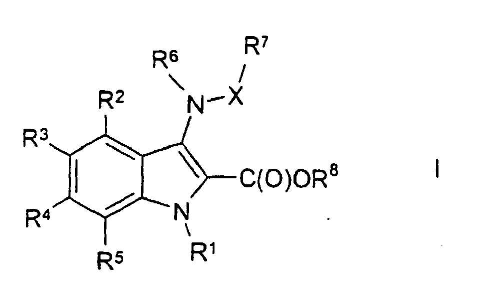 wherein
X represents a single bond, -C(O)- or -S(O)₂-;
R¹ represents an aryl group or a heteroaryl group, both of which groups are optionally substituted by one or more substituents selected from A;
   one of the groups R¹, R³, R⁴ and R⁵ represents an aryl group or a heteroaryl group (both of which are optionally substituted by one or more substituents selected from A) and:
   a) the other groups are independently selected from hydrogen, G¹, an aryl group, a heteroaryl group (which latter two groups are optionally substituted by one or more substituents selected from A), C₁₋₈ alkyl and a heterocycloalkyl group (which latter two groups are optionally substituted by one or more substituents selected from G¹ and/or Z¹); and/or
   b) any two other groups which are adjacent to each other are optionally linked to form; along with two atoms of the essential benzene ring in the compound of formula I, a 3- to 8-membered ring, optionally containing 1 to 3 heteroatoms and/or 1 to 3 double bonds, which ring is itself optionally substituted by one or more substituents selected from halo, -R⁸, -OR⁸ and =O;
R⁶, R⁷ and R⁸ independently represent, on each occasion when used above:
   I) hydrogen;
   II) an aryl group or a heteroaryl group, both of which are optionally substituted by one or more substituents selected from B;
   III) C₁₋₈ alkyl or a heterocycloalkyl group, both of which are optionally substituted by one or more substituents selected from G¹ and/or Z¹; or
      R⁶ and R⁷ may be linked together to form along with the N atom and X group to which R⁶ and R⁷ are respectively attached, a 3- to 8-membered ring, optionally containing 1 to 3 heteroatoms and/or 1 to 3 double bonds, which ring is optionally substituted by one or more substituents selected from G¹ and/or Z¹ ;
A represents, on each occasion when mentioned above:
   I) an aryl group or a heteroaryl group, both of which are optionally substituted by one or more substituents selected from B;
   II) C₁₋₈ alkyl or a heterocycloalkyl group, both of which are optionally substituted by one or more substituents selected from G¹ and/or Z¹;
   III) a G¹ group; or
   IV) two A substituents may be linked together to form, along with at least two (e.g. adjacent) atoms of the aryl or heteroaryl group to which the two A substituents are attached, a further 3- to 5-membered ring, which ring optionally contains 1 to 3 (e.g. I or 2) hetereoatoms and/or 1 to 2 (e.g. 1) double bonds, and which is optionally substituted by halo or C₁₋₈ alkyl, which latter group is optionally substituted by halo;
G¹ represents, on each occasion when mentioned above, halo, cyano, -N₃, -NO₂, -ONO₂ or -A¹ -R⁹;
   wherein A¹ represents a single bond or a spacer group selected from -C(O)A²-, -S(O)ₙA³-, -N(R¹⁰)A⁴- or -OA⁵-, in which:
   A² and A³ independently represent a single bond, -O-, -N(R¹⁰)- or -C(O)-;
   A⁴ and A⁵ independently represent a single bond, -C(O)-, -C(O)N(R¹⁰)-, -C(O)O-, -S(O)ₙ- or -S(O)ₙN(R¹⁰)-;
Z¹ represents, on each occasion when mentioned above, =O, =S, =NOR⁹, =NS(O)ₙN(R¹⁰)(R⁹), =NCN or =C(H)NO₂;
B represents, on each occasion when mentioned above:
   I) an aryl group or a heteroaryl group, both of which are optionally substituted by one or more substituents selected from G², methylenedioxy, difluoromethylenedioxy and/or dimethylmethylenedioxy;
   II) C₁₋₈ alkyl or a heterocycloalkyl group, both of which are optionally substituted by one or more substituents selected from G² and/or Z²;
   III) a G² group; or
   IV) methylenedioxy, difluoromethylenedioxy or dimethylmethylenedioxy;
G² represents, on each occasion when mentioned above, halo, cyano, -N₃, -NO, -ONO₂ or -A⁶-R¹¹;
   wherein A⁶ represents a single bond or a spacer group selected from -C(O)A⁷-, -S(O)ₙA⁸, -N(R¹²)A⁹- or -OA¹⁰-, in which:
   A⁷ and A⁸ independently represent a single bond, -O-, -N(R¹²)- or -C(O)-;
   - A⁹ and A¹⁰ independently represent a single bond, -C(O)-, -C(O)N(R¹²)-, -C(O)O-, -S(O)ₙ- or -S(O)ₙN(R¹²)-;
Z² represents, on each occasion when used above, =O, =S, =NOR¹¹, =NS(O)ₙN(R¹²)(R¹¹), =NCN or =C(H)NO₂;
R⁹, R¹⁰, R¹¹ and R¹² are independently selected from:
   i) hydrogen;
   ii) an aryl group or a heteroaryl group, both of which are optionally substituted by one or more substituents selected from G³, methylenedioxy, difluoromethylenedioxy and/or dimethylmethylenedioxy;
   iii) C₁₋₈ alkyl or a heterocycloalkyl group, both of which are optionally substituted by G³ and/or Z³; or
   any pair of R⁹ and R¹⁰, or R¹¹ and R¹², may, for example when present on the same or on adjacent atoms, be linked together to form with those, or other relevant, atoms a further 3- to 8-membered ring, optionally containing 1 to 3 heteroatoms and/or 1 to 3 double bonds, which ring is optionally substituted by one or more substituents selected from G³ and/or Z³;
G³ represents, on each occasion when mentioned above, halo, cyano, -N₃, -NO₂, -ONO₂ or -A¹¹-R¹³,
   wherein A¹¹ represents a single bond or a spacer group selected from -C(O)A¹²-, -S(O)ₙA¹³-, -N(R¹⁴)A¹⁴- or -OA¹⁵-, in which:
   A¹² and A¹³ independently represent a single bond, -O-, -N(R¹⁴)- or -C(O)-;
   A¹⁴ and A¹⁵ independently represent a single bond, -C(O)-, -C(O)N(R¹⁴)-, -C(O)O-, -S(O)ₙ- or -S(O)ₙN(R¹⁴)-;
Z³ represents, on each occasion when mentioned above, =O, =S, =NOR¹³, =NS(O)ₙN(R¹⁴)(R¹³), =NCN or =C(H)NO₂;
n represents, on each occasion when mentioned above, 1 or 2;
R¹³ and R¹⁴ are independently selected from:
   i) hydrogen;
   ii) C₁₋₆ alkyl or a heterocycloalkyl group, both of which groups are optionally substituted by one or more substituents selected from halo, C₁₋₄ alkyl, -N(R¹⁵)(R¹⁶), -O(R¹⁵) and =O; and
   iii) an aryl or heteroaryl group, both of which are optionally substituted by one or more substituents selected from halo, C₁₋₄ alkyl, -N(R¹⁵)(R¹⁶) and -O(R¹⁵); or
   any pair R¹³ and R¹⁴ may, for example when present on the same or on adjacent atoms, be linked together to form with those, or other relevant, atoms a further 3- to 8-membered ring, optionally containing 1 to 3 heteroatoms and/or 1 to 3 double bonds, which ring is optionally substituted by one or more substituents selected from halo, C₁₋₄ alkyl, -NC(R¹⁵)(R¹⁶), -O(R¹⁵) and =O;
R¹⁵ and R¹⁶ are independently selected from hydrogen and C₁₋₄ alkyl, which latter group is optionally substituted by one or more halo groups;
   or a pharmaceutically-acceptable salt thereof,
   which compounds and salts are referred to hereinafter as "the compounds of the invention".

Phannaceutically-acceptable salts include acid addition salts and base addition salts. Such salts may be formed by conventional means, for example by reaction of a free acid or a free base form of a compound of formula I with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

Compounds of the invention may contain double bonds and may thus exist as *E* (*entgegen*) and *Z* (*zusammen*) geometric isomers about each individual double bond. All such isomers and mixtures thereof are included within the scope of the invention.

Compounds of the invention may also exhibit tautomerism. All tautomeric forms and mixtures thereof are included within the scope of the invention.

Compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a 'chiral pool' method), by reaction of the appropriate starting material with a chiral auxiliary' which can subsequently, be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person. All stereoisomers and mixtures thereof are included within the scope of the invention.

Unless otherwise specified, C_{1-q} alkyl groups (where q is the upper limit of the range) defined herein may be straight-chain or, when there is a sufficient number (i.e. a minimum of two or three, as appropriate) of carbon atoms, be branched-chain, and/or cyclic (so forming a C_{3-q} cycloalkyl group). C_{3-q} cycloalkyl groups that may be mentioned include monocyclic or bicyclic alkyl groups, which cycloalkyl groups may further be bridged. Further, when there is a sufficient number (i.e. a minimum of four) of carbon atoms, such groups may also be part cyclic. Such alkyl groups may also be saturated or, when there is a sufficient number (i.e. a minimum of two) of carbon atoms, be unsaturated (forming, for example, a C_{3-q} cycloalkenyl, a C₈ cycloalkynyl or, more particularly, a C_{2-q} alkenyl or a C_{2-q} alkynyl group). Further, in the case where the substituent is another cyclic compound, then the cyclic substituent may be attached through a single atom on the cycloalkyl group, fonning a so-called "spiro"-compound.

The term "halo", when used herein, includes fluoro, chloro, bromo and iodo.

Heterocycloalkyl groups that may be mentioned include those in which at least .one (e.g. one to four) of the atoms in the ring system is other than carbon (i.e. a heteroatom), and in which the total number of atoms in the ring system is between three and twelve (e.g. between five and ten). Further, such heterocycloalkyl groups may be saturated or unsaturated containing one or more double and/or triple bonds, forming for example a C_{2-q} (e.g. C_{3-q}) heterocycloalkenyl (where q is the upper limit of the range) or a C_{3-q} heterocycloalkynyl group. C_{2-q} heterocycloalkyl groups that may be mentioned include aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, morpholinyl, oxetanyl, oxiranyl, piperazinyl, piperidinyl, pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuranyl, tetrahydropyridyl, thietanyl, thiiranyl, thiolanyl, thiomorpholinyl, trithianyl (including 1,3,5-trithianyl), tropanyl and the like. Other heterocycloalkyl groups that may be mentioned include 7-azabicyclo[2.2.1]heptanyl, 6-azabicyclo[3.1.1]heptanyl, 6-azabicyclo-[3.2.1]octanyl, 8-azabicyclo[3.2.1]-octanyl, 7-oxabicyclo[2.2.1]heptanyl and 6-oxabicyclo[3.2.1]octanyl. Heterocycloalkyl groups that may be mentioned include monocyclic and bicyclic heterocycloalkyl groups, which groups may further be bridged. Substituents on heterocycloalkyl groups may, where appropriate, be located on any atom in the ring system including a heteroatom. Further, in the case where the other substituent is another cyclic compound, then the cyclic compound may be attached through a single atom on the heterocycloalkyl group, forming a so-called "spiro"-compound. The point of attachment of heterocycloalkyl groups may be *via* any atom in the ring system including (where appropriate) a heteroatom (such as a nitrogen atom), or an atom on any fused carbocyclic ring that may be present as part of the ring system. Heterocycloalkyl groups may also be in the *N*- or *S*- oxidised form.

For the avoidance of doubt, the term "bicyclic", when employed in the context of cycloalkyl and heterocycloalkyl groups refers to such groups in which the second ring is formed between two adjacent atoms of the first ring. The term "bridged", when employed in the context of cycloalkyl or heterocycloalkyl groups refers to monocyclic or bicyclic groups in which two non-adjacent atoms are linked by either an alkylene or heteroalkylene chain (as appropriate).

Aryl groups that may be mentioned include C₆₋₁₃ (e.g. C₆₋₁₀) aryl groups. Such groups may be monocyclic or bicyclic and have between 6 and 13 (e.g. 10) ring carbon atoms, in which at least one ring is aromatic. C₆₋₁₃ aryl groups include phenyl, naphthyl and the like, such as fluorenyl and, more particularly, 1,2,3,4-tetrahydronaphthyl, indanyl, and indenyl. The point of attachment of aryl groups may be *via* any atom of the ring system. However, when aryl groups are bicyclic or tricyclic, they are preferably linked to the rest of the molecule visa an aromatic ring.

Heteroaryl groups that may be mentioned include those which have between 5 and 10 members. Such groups may be monocyclic, bicyclic or tricyclic, provided that at least one of the rings is aromatic and wherein at least one (e.g. one to four) of the atoms in the ring system is other than carbon (i.e. a heteroatom). Heterocyclic groups that may be mentioned include acridinyl, benzimidazolyl, benzodioxanyl, benzodioxepinyl, benzodioxolyl (including 1,3-benzodioxolyl), benzofuranyl, benzofurazanyl, benzothiazolyl (including 2,1,3-benzothiazolyl), benzoxadiazolyl (including 2,1,3-benzoxadiazolyl), benzoxazinyl (including 3.4-dihydro-2*H*-1,4-benzoxazinyl), benzoxazolyl, benzimidazolyl, benzomorpholinyl, benzoselenadiazolyl (including 2,1,3-benzoselenadiazolyl), benzothienyl, carbazolyl, chromanyl, cinnolinyl, furanyl, imidazolyl, imidazo[1,2-*a*]pyridyl, indazolyl, indolinyl, indolyl, isobenzofuranyl, isochromanyl, isoindolinyl, isoindolyl, isoquinolinyl, isothiaziolyl, isoxazolyl, naphthyridinyl (including 1,5-naphthyridinyl and 1,8-naphthyridinyl), oxadiazolyl (including 1,2,3-oxadiazolyl, 1,2,4-oxadiazolyl and 1,3,4-oxadiazolyl)_{,} oxazolyl phenazinyl, phenothiazinyl, phthalazinyl, pteridinyl, purinyl, pyrazinyl, pyrazolyl, pyridazinyl, pyridyl, pyrimidinyl, pyrrolyl, quinazolinyl, quinolinyl, quinolizinyl, quinoxalinyl, tetrahydroisoquinolinyl (including 1,2,3_{,}4-tetrahydroisoquinolinyl and 5,6,7,8-tetrahydroisoquinolinyl), tetrahydroquinolinyl (including 1,2,3,4-tetrahydroquinolinyl and 5,6,7,8-tetrahydroquinolinyl), tetrazolyl, thiadiazolyl (including 1,2,3-thiadiazolyl, 1.2,4-thiadiazolyl and 1,3,4-thiadiazolyl)_{;} thiazolyl, thiochromanyl, thienyl, triazolyl (including 1,2,3-triazolyl, 1,2,4-triazolyl and 1,3,4-triazolyl) and the like. Substituents on heteroaryl groups may, where appropriate, be located on any atom in the ring system including a heteroatom. The point of attachment of heteroaryl groups may be via any atom in the ring system including (where appropriate) a heteroatom (such as a nitrogen atom), or an atom on any fused carbocyclic ring that may be present as part of the ring system. However, when heteroaryl groups are bicyclic or tricyclic, they are preferably linked to the rest of the molecule *via* an aromatic ring. Heteroaryl groups may also be in the *N*- or *S*- oxidised form.

Heteroatoms that may be mentioned include phosphorus, silicon, boron, tellurium, preferably, selenium and, more preferably oxygen, nitrogen and/or sulfur.

For the avoidance of doubt, optionally substituted methylenedioxy groups, when attached to a ring system, are formed between any two adjacent atoms of the ring system.

For the avoidance of doubt, in cases in which the identity of two or more substituents in a compound of the invention may be the same, the actual identities of the respective substituents are not in any way interdependent. For example, in the situation in which R¹, and any one of R² to R⁵, both represent aryl groups substituted by one or more C₁₋₈ alkyl groups, the alkyl groups in question may be the same or different. Similarly, when groups are substituted by more than one substituent as defined herein, the identities of those individual substituents are not to be regarded as being interdependent. For example, when R¹ represents e.g. an aryl group substituted by G¹ in addition to, for example, C₁₋₈ alkyl, which latter group is substituted by G¹, the identities of the two G¹ groups are not to be regarded as being interdependent.

Compounds of the invention that may be mentioned include those in which:
A² and A³ independently represent a single bond, -O- or -N(R¹⁰)-;
Z¹ represents, on each occasion when mentioned above, =O, =NOR⁹, =NS(O)ₙN(R¹⁰)(R⁹), =NCN or =C(H)NO₂;
A⁷ and A⁸ independently represent a single bond, -O- or -N(R¹²)-;
Z² represents, on each occasion when mentioned above, =O, =NOR¹¹, =NS(O)ₙN(R¹²)(R¹¹), =NCN or =C(H)NO²;
A¹² and A¹³ independently represent a single bond, -O- or -N(R¹⁴)-; and/or
Z³ represents, on each occasion when mentioned above, =O, =NOR¹³, =NS(O)ₙN(R¹⁴)(R¹³), =NCN or =C(H)NO₂.

Preferred compounds of the invention include those in which:
X represents a single bond or -C(O)-;
G¹ represents halo, cyano, -N₃, -NO₂ or -A¹-R⁹;
A⁴ and A⁵ independently represent a single bond, -C(O)-, -C(O)N(R¹⁰)- or -C(O)O-;
Z¹ represents =NOR⁹, =NCN or, preferably, =O;
G² represents cyano, -N₃ or, more preferably, halo, -NO₂ or -A⁶-R¹¹;
A⁶ represents -N(R¹²)A⁹- or -OA¹⁰-;
A⁹ represents -C(O)N(R¹²)-, -C(O)O- or, more preferably, a single bond or -C(O)-;
A¹⁰ represents A⁹ and, preferably, a single bond;
Z² represents =NOR¹¹ or =NCN or, more preferably, =O;
G³ represents halo, -NO₂ or -A¹¹-R¹³;
A¹¹ represents -N(R¹⁴)- or -O-;
Z³ represents =O;
n represents 2;
when either of R¹³ and R¹⁴ represent optionally substituted C₁₋₆ alkyl, the optional substituent is one or more halo groups;
when either of R¹⁵ and R¹⁶ represent optionally substituted C₁₋₄ alkyl, the optional substituent is one or more fluoro groups.

Preferred compounds of the invention include those in which R¹ and (when they represent an aryl or heteroaryl group) R², R³, R⁴ and/or R⁵ represent an optionally substituted phenyl, naphthyl, pyrrolyl, furanyl, thienyl, pyrazolyl, imidazolyl (e.g 1-imidazolyl, 2-imidazolyl or 4-imidazolyl), oxazolyl, isoxazolyl, thiazolyl, pyridyl (e.g. 2-pyridyl, 3-pyridyl or 4-pyridyl), indazolyl, indolyl, indolinyl, isoindolinyl, quinolinyl, 1,2,3,4-tetrahydroquinolinyl, isoquinolinyl, 1,2,3,4-tetrahydroisoquinolinyl, quinolizinyl, benzofuranyl, isobenzofuranyl, chromanyl, benzothienyl, pyridazinyl, pyrimidinyl, pyrazinyl, indazolyl, benzimidazolyl, quinazolinyl, quinoxalinyl, 1,3-benzodioxolyl, benzothiazolyl, and/or benzodioxanyl, group. Other groups that may be mentioned include optionally substituted 5,6,7,8-tetrahydroquinolinyl, 5,6,7,8-tetrahydroisoquinolinyl and tetrazolyl. Particularly preferred values include optionally substituted phenyl and pyridyl groups.

More preferred compounds include those in which:
R⁶ represents H or optionally substituted C₁₋₆ alkyl;
R⁷ represents optionally substituted heteroaryl or, more preferably, optionally substituted C₁₋₆ alkyl or optionally substituted aryl; or
R⁶ and R⁷ are optionally linked as hereinbefore defined.

Optional substituents on R¹, R², R³, R⁴, R⁵, R⁶ and R⁷ groups are preferably selected from cyano, and, more preferably from:
halo (e.g. fluoro, chloro or bromo);
C₁₋₆ alkyl, which alkyl group may be linear or branched (e.g. C₁₋₄ alkyl (including methyl, ethyl, *n*-propyl, isopropyl, *n*-butyl, isobutyl or *t*-butyl), *n*-pentyl, isopentyl; *n*-hexyl or isohexyl), cyclic (e.g. cyclopropyl, cyclobutyl, cyclopentyl or cyclohexyl), part-cyclic (e.g. cyclopropylmethyl), unsaturated (e.g. 1-propenyl, 2-propenyl, 1-butenyl, 2-butenyl, 3-butenyl, 1-pentenyl, 2-pentenyl, 4-pentenyl or 5-hexenyl) and/or optionally substituted with one or more halo (e.g. fluoro) group (so forming, for example, fluoromethyl, difluoromethyl or trifluoromethyl); and -OR¹⁷;
wherein R¹⁷ represents, H or C₁₋₆ alkyl, such as methyl, ethyl, *n*-propyl, isopropyl, *n*-butyl, isobutyl or *t*-butyl (which alkyl, groups are optionally substituted by one or more halo (e.g. fluoro) groups).

Preferred values of R⁸ are C₁₋₄ alkyl and, particularly, hydrogen.

More preferred compounds include those in which:
R¹ represents an aryl group, such as a phenyl group, or a heteroaryl group such as a pyridyl group, both of which groups are optionally substituted by one or two A groups;
R³ and R⁴ independently represent G¹ or, more preferably, H, an aryl group, such as phenyl, or a heteroaryl group such as pyridyl, both of which groups are optionally substituted by one or two A groups;
   at least one of R³ and R⁴ represents optionally substituted aryl or heteroaryl, and up to one other represents G¹ or, more preferably, hydrogen;
   when R³ or R⁴ represents an aryl or heteroaryl group, then the other substituents on the essential benzene ring of the compound of formula I (i.e. R², R⁵ and R³ or R⁴ (as appropriate)) independently represent hydrogen or G¹ (e.g. halo (such as chloro), cyano, methyl, methoxy, trifluoromethyl or trifluoromethoxy);
R⁶ represents H or C₁₋₄ alkyl, which alkyl group is optionally substituted by one or two G¹ groups;
R⁷ represents C₁₋₄ alkyl, which group is optionally substituted by one or two G¹ groups, an aryl group, such as a phenyl group, or a heteroaryl group, such as a pyridyl group, which latter two groups are optionally substituted by one or two B groups; or
R⁶ and R⁷ are linked to form, together with the nitrogen atom and X group to which they are respectively attached, a 5- to 6-membered ring, optionally containing 1 to 2 heteroatoms;
A represents G¹ ;
G¹ represents halo (e.g. chloro) or -A¹-R⁹;
A¹ represents a single bond, -OA⁵- or -N(R¹⁰)A⁴-;
A⁴ and A⁵ independently represent a single bond;
B represents G²;
G² represents halo or -A⁶-R¹¹;
A⁶ represents -O-;
R⁹ represents C₁₋₆ (e.g. C₁₋₃) alkyl, which group is optionally substituted by one or more G³ groups, or an aryl group, such as a phenyl, or a heteroaryl group, such as a pyridyl group;
R¹⁰ represents C₁₋₂ alkyl;
R¹¹ represents C₁₋₂ alkyl optionally substituted by one or more G³ groups;
G³ represents halo (especially fluoro);

Especially preferred compounds of the invention are wherein:
R⁸ represents hydrogen;
R¹ represents a phenyl group, substituted, for example in the 3- or, preferably, 4-position by a single -A¹-R⁹ group. In such instances, A¹ may represent -OA⁵-, in which A⁵ is as hereinbefore defined and is preferably a single bond. R⁹ may, in such instances, represent C₁₋₅ (e.g. C₁₋₄) alkyl, such as cyclic C₃₋₅ alkyl (e.g. cyclopenyl) or, preferably, optionally branched propyl, so forming, for example, a 4-cyclopentoxyphenyl or, more preferably, a 4-isopropoxyphenyl group;
R² represents halo, cyano, C₁₋₃ aryl, C₁₋₃ alkoxy (which latter two groups are optionally substituted by one or more halo (e.g. fluoro) groups) or, preferably, H;
R³ represents a phenyl group, substituted; for example in the 3- or, preferably, 4-position by a single -A¹-R⁹ group. In such instances, A¹ may represent a single bond or -OA⁵-. When A¹ represents -OA⁵-, A⁵ is as hereinbefore defined and is preferably a single bond and R⁹ may represent C₁₋₄ alkyl, such as branched propyl. When A¹ represents a single bond, R⁹ may represent a C₁₋₄ (e.g. C₁₋₂) alkyl group, such as an optionally branched butyl group (e.g. *t*-butyl) or, more particularly, a methyl group, optionally substituted by one or more G³ groups, in which G³ represents halo (especially fluoro). Thus R¹ may represent a 4-*tert*-butylphenyl or, more particularly, a 4-isopropoxyphenyl or 4-trifluoromethylphenyl group;
R³ may alternatively represent a pyridyl group (e.g. a 2-pyridyl group), optionally substituted, for example in the *meta* or, preferably, *para* position relative to the point of attachment of R³ to the indole ring, by a single -A¹-R⁹ group. In such instances, A¹ preferably represents a single bond and R⁹ represents C₁₋₂ alkyl (e.g. methyl) optionally substituted by one or more G³ groups, in which G³ represents fluoro, so forming, for example, a 5-trifluoromethylpyrid-2-yl group;
R⁴ and R⁵ independently represent halo, C₁₋₃ alkyl, C₁₋₃ alkoxy (which latter two groups are optionally substituted by one or more halo (e.g. fluoro) groups) or, preferably, H;
R⁶ may represent H or a C₁₋₃ alkyl group, such as a methyl or n-propyl group, optionally substituted, for example at the terminal position, by a G¹ group. In such instances, G¹ may represent -A¹-R⁹, in which A¹ preferably represents a single bond and R⁹ preferably represents an aryl group, such as phenyl, or a heteroaryl group, such as pyridyl (especially 3-pyridyl). Thus, R⁶ may also represent a 3-phenylpropyl, a pyrid-3-ylmethyl or a methyl group;
R⁷ may represent C₁₋₃ alkyl, such as methyl or *n*-propyl, optionally substituted, for example, at the terminal position, by a G¹ group, an aryl group, such as a phenyl group or a heteroaryl group, such as a pyridyl group, which latter two groups are optionally substituted. For example, the phenyl group may be substituted in the 3- or, preferably, 4-position, by a B group. In the instance wherein the substituent is G', G¹ may represent -A¹-R⁹, in which A¹ preferably represents a -N(R¹⁰)A⁴ group, in which A⁴ preferably represents a single bond, and R¹⁰ and R¹¹ are each, independently, C₁₋₂ alkyl, such as methyl. In the instance wherein the substituents is B, B may represent a G² group, in which G² is preferably a halo group (such as chloro) or a -A⁶-R¹¹ group. In such instances, A⁶ preferably represents -O- and R¹¹ preferably represents C₁₋₂ alkyl, such as methyl, optionally substituted by one or more G³ groups, in which G³ represents halo (especially fluoro). Thus R⁷ may represent a 3-pyridyl or, more preferably, a dimethylaminopropyl, (4-trifluoromethoxy)phenyl, a 4-chlorophenyl, or a methyl group;
   when R⁶ and R⁷ are linked together with the nitrogen atom and X group, to which they are respectively attached, then X is -C(O)- or, preferably, a single bond and the ring formed is preferably a 5- to 6-membered ring, optionally containing a further heteroatom (e.g. an oxygen heteroatom) so forming, for example, a pyrrolidinone (e.g. a 1-pyrrolidinone) group or, more preferably, a morpholinyl group (e.g. a 4-morpholinyl group).

Particularly preferred compounds of the invention include those of the example described hereinafter.

Compounds of the invention may be made in accordance with techniques that are well known to those skilled in the art, for example as described hereinafter.

According to a further aspect of the invention there is provided a process for the preparation of a compound of formula 1, which process comprises:
(i) reaction of a compound of formula II, 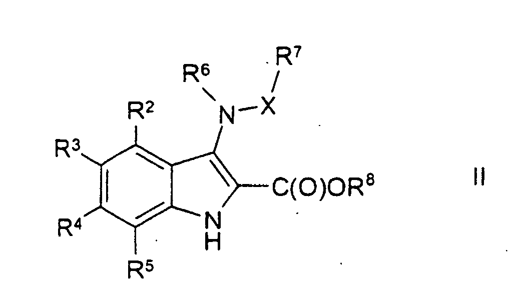 wherein X, R², R³, R⁴, R⁵, R⁶, R⁷ and R⁸ are as hereinbefore defined, With a compound of formula III,

   R¹L¹ III

   wherein L¹ represents a suitable leaving group such as chloro, bromo, iodo, a sulfonate group (e.g. -OS(O)₂CF₃, -OS(O)₂CH₃, -OS(O)₂PhMe or a nonaflate) or -B(OH)₂ and R¹ is as hereinbefore defined, for example optionally in the presence of an appropriate metal catalyst (or a salt or complex thereof) such as Cu, Cu(OAc)₂, CuI (or CuI/diamine complex), Pd(OAc)₂, Pd₂(dba)₃ or NiCl₂ and an optional additive such as Ph₃P, 2,2'-bis(diphenylphosphino)-1,1'-binaphthyl, xantphos, NaI or an appropriate crown ether such as 18-crown-6-benzene, in the presence of an appropriate base such as NaH, Et₃N, pyridine, *N,N*'-dimethylethylenediamine, Na₂CO₃, K₂CO₃, K₃PO₄, Cs₂CO₃, *t*-BuONa or *t-*BuOK (or a mixture thereof), in a suitable solvent (e.g. dichloromethane, dioxane, toluene, ethanol, isopropanol, dimethylformamide, ethylene glycol, ethylene glycol dimethyl ether, water, dimethylsulfoxide, acetonitrile, dimethylacetamide, *N-*methylpyrrolidinone, tetrahydrofuran or a mixture thereof) or in the absence of an additional solvent when the reagent may itself act as a solvent (e.g. when R¹ represents phenyl and L¹ represents bromo, i.e. bromobenzene). This reaction may be carried out at room temperature or above (e.g. at a high temperature, such as the reflux temperature of the solvent system that is employed) or using microwave irradiation;
(ii) reaction of a compound of formula IV, 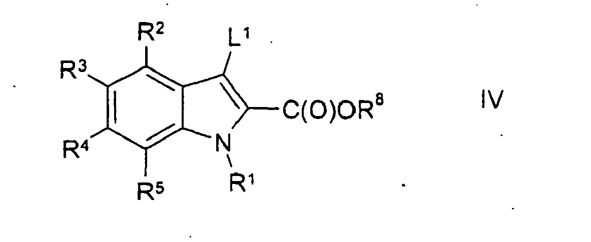 wherein L¹, R¹, R², R³, R⁴, R⁵ and R⁸ are as hereinbefore defined, with a compound of formula V,

   HN(R⁶)X(R⁷ V

   wherein X, R⁶ and R⁷ are as hereinbefore defined for example under reaction conditions as hereinbefore defined in respect of process step (i);
(iii) reaction of a compound of formula VI, 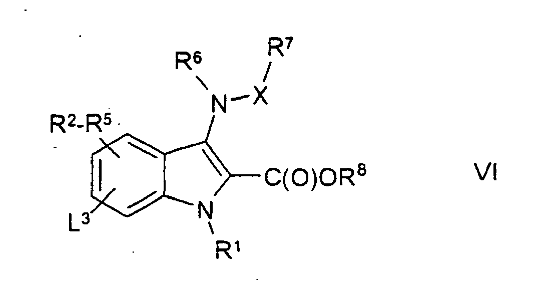 wherein L³ represents L¹ or L², in which L² represents a suitable leaving group such as chloro, bromo, iodo, -B(OH)₂ or a protected derivative thereof, for example a 4,4,5,5-tetramethyl-1,3,2-dioxaborolan-2-yl group, 9-borabicyclo[3.3.1]nonane (9-BBN), -Sn(alkyl)₃ (e.g. -SnMe₃ or -SnBu₃), or a similar group known to the skilled person, and wherein L³ is attached to one or more of the carbon atoms of the benzenoid ring of the indole, and
   wherein the remaining positions of the benzenoid ring are substituted with 1 to 3 (depending on the number of L³ substituents) R²-R⁵ substituents, R²-R⁵ represents any one of the substituents, i.e. R², R³, R⁴ and R⁵, that are already present in that ring (as appropriate), and X, L¹, R¹, R², R³, R⁴, R⁵, R⁶, R⁷ and R⁸ are as hereinbefore defined, with a compound of formula VII,

   R¹⁸L⁴ VII

   wherein R¹⁸ represents R², R³, R⁴ or R⁵ (as appropriate), and L⁴ represents L¹ (when L³ is L²) or L² (when L³ is L¹) as hereinbefore defined. The skilled person will appreciate that L¹ and L² will be mutually compatible. This reaction may be performed, for example in the presence of a suitable catalyst system, e.g. a metal (or a salt or complex thereof) such as CuI, PdCl₂, Pd/C, Pd(OAc)₂, Pd(Ph₃P)₂Cl₂, Pd(Ph₃P)_{4,} Pd₂(dba)₃ or NiCl₂ and an additive such as *t*-Bu₃P, (C₆H₁₁)₃P, Ph₃P, AsPh₃, P(*o*-Tol)₃, 1,2-bis(diphenylphosphino)ethane, 2,2'-bis(di-*tert*-butylphosphino)-1,1'-biphenyl, 2,2'-bis(diphenylphosphino)-1,1'-binaphthyl, 1,1'-bis(diphenylphosphinoferrocene), 1,3-bis(diphenyl-phosphino)propane or xantphos, together with a suitable base such as, Na₂CO₃, K₃PO₄, Cs₂CO₃, KOH, NaOH, K₂CO₃, CsF, Et₃N, (*i*-Pr)₂NEt, *t*-BuONa or *t*-BuOK (or mixtures thereof) in a suitable solvent such as dioxane, toluene, ethanol, dimethylformamide, ethylene glycol dimethyl ether, water, dimethylsulfoxide, acetonitrile, dimethylacetamide, N-methylpyrrolidinone, tetrahydrofuran or mixtures thereof. The reaction may also be carried out for example at room temperature or above (e.g. at a high temperature such as the reflux temperature of the solvent system) or using microwave irradiation. The skilled person will appreciate that when L³ or L⁴ (of the compounds of formulae VI and VII, respectively, represent halo, such compounds may first be activated by:
   (I) forming the corresponding Grignard reagent under standard conditions known to those skilled in the art (e.g. employing magnesium or a suitable reagent such as a mixture of C₁₋₆ alkyl-Mg-halide and ZnCl₂ or LiCl), followed by reaction with a compound of formula VI or VII (as appropriate), optionally in the presence of a catalyst (e.g. FeCl₃) under conditions known to those skilled in the art; or
   (II) forming the corresponding lithiated compound under halogen-lithium exchange reaction conditions known to those skilled in the art (e.g. employing *n*-BuLi or *t*-BuLi in the presence of a suitable solvent (e.g. a polar aprotic solvent, such as THF)), followed by reaction with a compound of formula VI or VII (as appropriate).

   The skilled person will also appreciate that the magnesium of the Grignard reagent or the lithium of the lithiated species may be exchanged for a different metal (i.e. a transmetallation reaction may be performed), for example to zinc (e.g. using ZnCl₂) and the intermediate so formed may then be subjected to reaction with a compound of formula VI or VII (as appropriate) under conditions known to those skilled in the art, for example such as those described above;
(iv) reaction of a compound of formula VIII, 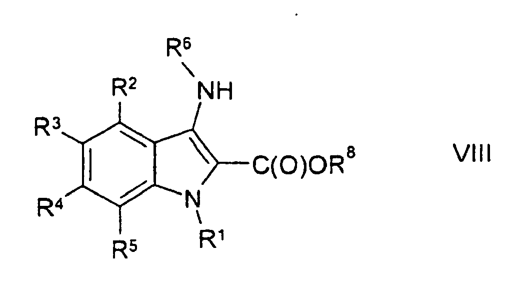 wherein R¹, R², R³, R⁴, R⁵, R⁶ and R⁸ are as hereinbefore defined, with a compound of formula IX,

   R⁷XL¹ IX

   wherein X, R⁷ and L¹ are as hereinbefore defined, for example at around room temperature, below room temperature (e.g. at 0°C) or above room temperature (e.g. up to 60-70°C) optionally in the presence of a suitable base (e.g. pyrrolidinopyridine, pyridine; triethylamine, tributylamine, trimethylamine, dimethylaminopyridine, diisopropylamine, 1,8-diazabicyclo[5.4.0]undec-7-ene, sodium hydroxide, or mixtures thereof) and an appropriate solvent (e.g. pyridine, dichloromethane, chloroform, tetrahydrofuran, dimethylformamide, trifluoromethylbenzene or acetonitrile. This reaction may be performed under an inert atmosphere (e.g. under Ar); or
(v) for compounds of formula I wherein X represents a single bond and R⁷ is a C₁₋₈ alkyl group, reduction of a compound of formula I, wherein X represents -C(O)- and R⁷ represents H or a C₁₋₇ alkyl group, in the presence of a suitable reducing agent. A suitable reducing agent may be an appropriate reagent that reduces the amide group to the amine group in the presence of other functional groups (for example an ester or a carboxylic acid). Suitable reducing agents include borane and other reagents known to the skilled person, under reaction conditions known to the skilled person.

Compounds of formula II may be prepared by:
(a) reaction of a compound of formula X, 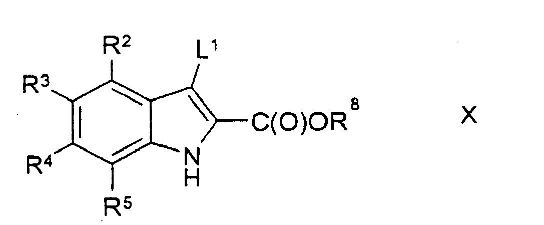 wherein L¹, R², R³, R⁴, R⁵ and R⁸ are as hereinbefore defined, with a compound of formula V as hereinbefore defined, for example under conditions such as those described hereinbefore in respect of preparation of compounds of formula I (process step (i)) above;
(b) reaction of a compound of formula XI, 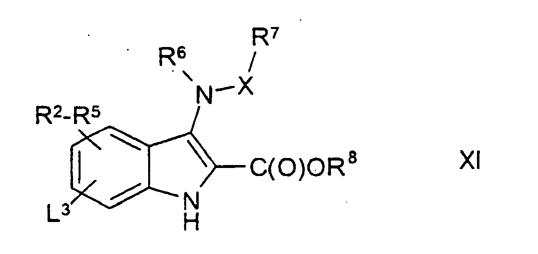 wherein X, L³, R²-R⁵, R⁶, R⁷ and R⁸ are as hereinbefore defined with a compound of formula VII as hereinbefore defined, for example under conditions such as those described hereinbefore in respect of preparation of compounds of formula I (process step (iii)) above; or
(c) reaction of a compound of formula XII, 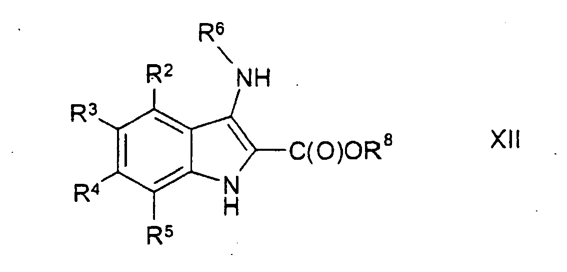 wherein R², R³, R⁴, R⁵, R⁶ and R⁸ are as hereinbefore defined, with a compound of formula IX, as hereinbefore defined, for example under reaction conditions such as those described hereinbefore in respect of preparation of compounds of formula I (process step (iv)) above.

Compounds of formula IV may be prepared by:
(a) reaction of a compound of formula X as hereinbefore described with a compound of formula XIII,

   R¹L² XIII

   wherein R¹ and L² are as hereinbefore defined, for example under conditions such as those described hereinbefore in respect of preparation of compounds of formula I (process step (iii)) above;
(b) reaction of a compound of formula X as hereinbefore described with a compound of formula III, as hereinbefore defined, for example under reaction conditions such as those described hereinbefore in respect of preparation of compounds of formula I (process step (i)) above; or
(c) for compounds of formula IV wherein L¹ represents a sulfonate group, reaction of a compound of formula XIV, 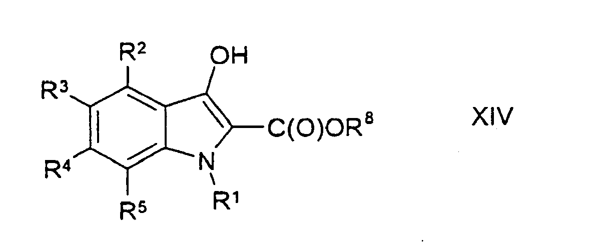
wherein R¹, R², R³, R⁴, R⁵ and R⁸ are as hereinbefore defined, with an appropriate reagent for the conversion of the hydroxyl group to the sulfonate group (e.g. tosyl chloride, mesyl chloride, triflic anhydride and the like) under conditions known to those skilled in the art.

Compounds of formula VI may be prepared by reaction of a compound of formula XI as hereinbefore defined, with a compound of formula III as hereinbefore defined, for example under reaction conditions such as those described hereinbefore in respect of preparation of compounds of formula I (process step (i)) above.

Compounds of formula VI in which L³ represents L² may be prepared by reaction of a compound of formula VI in which L³ represents L¹, with an appropriate reagent for the conversion of the L¹ group to the L² group. This conversion may be performed by methods known to those skilled in the art, for example:
i) compounds of formula VI, in which L³ is 4,4,5,-tetramethyl-1,3,2-dioxaborolan-2-yl may be prepared by reaction of the reagent bis(pinacolato)diboron with a compound of formula VI in which L³ represents L¹, for example under reaction conditions such as those described hereinbefore in respect of preparation of compounds of formula I (process step (iii)) above;
ii) compounds of formula VI, in which L³ represents -B(OH)₂ may be prepared by reaction of a corresponding compound of formula VI in which L³ represents halo by reaction with, for example, boronic acid or a protected derivative thereof (e.g. bis(pinacolato)diboron or triethyl borate) followed by (if necessary) deprotection under standard conditions. The skilled person will appreciate that the compound of formula VI in which L³ represents halo may first need to be converted to the corresponding Grignard reagent, or another metal (e.g. *via* a transmetallation reaction), for example under conditions such as those described in respect of preparation of compounds of formula I (process step (iii)) above; or
iii) compounds of formula VI in which L³ represents a halo group may be prepared by reaction of a corresponding compound of formula VI in which L³ represents a different halo group, for example employing a suitable source of halide ions such as those described hereinafter in respect of preparation of compounds of formula X (process (a)) under conditions known to those skilled in the art. For example, conversion of a bromo group to an iodo group may be performed in the presence of NaI, optionally in the presence of a suitable catalyst (e.g. CuI) and/or a catalytic amount of base (e.g. *N'N*,-dimethyl-1,2-diaminoethane) in the presence of a suitable solvent such as one described hereinbefore in respect of preparation of compounds of formula I (process step (i)).

Conversions of the L⁴ group and the L³ group in the compounds of formulae VII and XI, respectively, may be performed in a similar manner to that described above in respect of converting the L³ group in compounds of formula VI.

Compounds of formula X may be prepared by standard techniques. For example:
(a) compounds of formula X, wherein L¹ represents halo (e.g. bromo or iodo), may be prepared by reaction of a compound of formula XV, 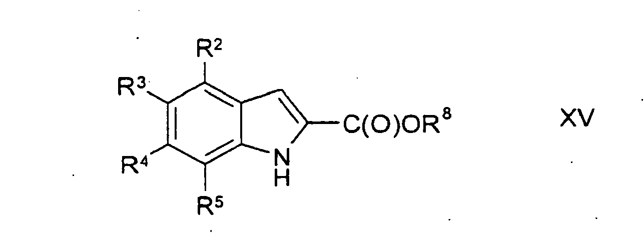 wherein R², R³, R⁴, R⁵ and R⁸ are as hereinbefore defined, with a reagent, or mixture of reagents known to be a source of halide (e.g. bromide or iodide) ions. For example, for bromide ions, *N-*bromosuccinimide may be employed, for iodide ions, iodine or a mixture of NaI and *N-*chlorosuccinimide may be employed, for chloride ions, *N-*chlorosuccinimide may be employed and for fluoride ions, 1-(chloromethyl)-4- fluoro-1,4-diazoniabicyclo[2.2.2]octane bis(tetrafluoroborate) may be employed. This reaction may be carried out in a suitable solvent (e.g. acetone, benzene or dioxane) under conditions known to the skilled person;
(b) by reaction of a compound of formula XVI, 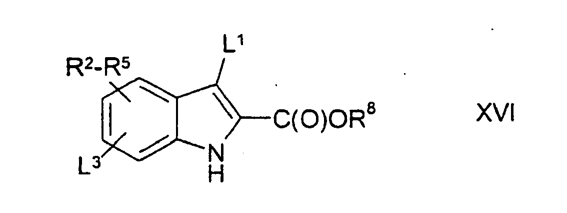 wherein L¹, L³, R², R³, R⁴, R⁵ and R⁸ are as hereinbefore defined with a compound of formula VII as hereinbefore defined, for example under reaction conditions such as those described hereinbefore in respect of preparation of compounds of formula I (process step (iii)) above; or
(c) compounds of formula X, wherein L¹ represents a sulfonate group may be prepared by reaction of a compound of formula XVII, 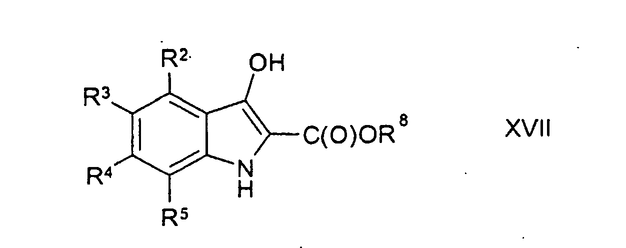 wherein R², R³, R⁴, R⁵ and R⁸ are as hereinbefore defined, with an appropriate reagent for the conversion of the hydroxyl group to a sulfonate group as described hereinbefore.

Compounds of formula XII may be prepared for example by reaction of a compound of formula X, as hereinbefore defined, with a compound of formula XVIII,

H₂NR⁶ XVIII

wherein R⁶ is as hereinbefore defined, for example under reaction conditions such as those described hereinbefore in respect of preparation of compounds of formula I (process step (ii)) above;

Compounds of formula XII, wherein R⁶ represents hydrogen may be prepared for example by an aromatic nitration reaction performed on a compound of formula XV, as hereinbefore defined, followed by reduction of the nitro group to the amino group. Both reactions may be performed under conditions known to the skilled person.

Compounds of formulae III, V, VII, VIII, IX, XI, XIII, XIV, XV, XVI, XVII and XVIII are either commercially available, are known in the literature, or may be obtained either by analogy with the processes described herein, or by conventional synthetic procedures, in accordance with standard techniques, from available starting materials using appropriate reagents and reaction conditions. In this respect, the skilled person may refer to *inter alia "*Comprehensive Organic Synthesis" by B. M. Trost and 1. Fleming, Pergamon Press, 1991.

Indoles of formulae II, IV, VI, VIII, X, XI, XII, XIV, XV, XVI and XVII may also be prepared with reference to a standard heterocyclic chemistry textbook (e.g. "Heterocyclic Chemistry" by J. A. Joule, K. Mills and G. F. Smith, 3rd edition, published by Chapman & Hall or "Comprehensive Heterocyclic Chemistry II" by A. R. Katritzky, C. W. Rees and E. F. V. Scriven, Pergamon Press, 1996) and/or made according to the following general procedures.

For example compounds of formulae II, XI, XII and XV may be prepared by reaction of a compound of formula XIX, 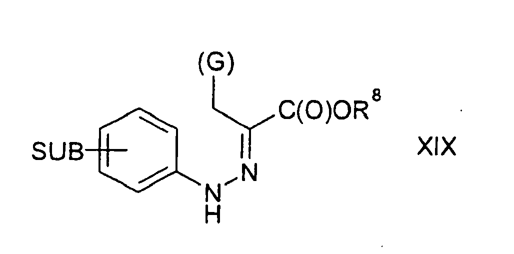 wherein SUB represents the substitution pattern that is present in the compound of formula II, XI, XII or XV to be formed, (G) represents either a -N(R⁶)C(O)R⁷ group (as required for formation of compounds of formulae II and XI), a -N(R⁶)H group (as required for formation of compounds of formula XII) or hydrogen (as required for formation of compounds of formula XV) and R⁸ is as hereinbefore defined, under Fischer indole synthesis conditions known to the person skilled in the art.

Compounds of formula XV may alternatively be prepared by reaction of a compound of formula XX, 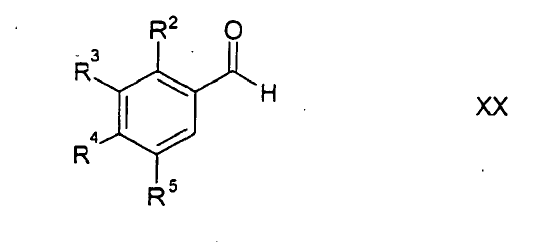 wherein R², R³, R⁴ and R³ are as hereinbefore defined with a compound of formula XXI,

N₃CH₂C(O)OR⁸ XXI

wherein R⁸ is as hereinbefore defined, and preferably does not represent hydrogen, under conditions known to the person skilled in the art (i.e. conditions to induce a condensation reaction, followed by a thermally induced cyclisation).

Compounds of formulae XIV and XVII may be prepared by reaction of a compound of formula XXII, 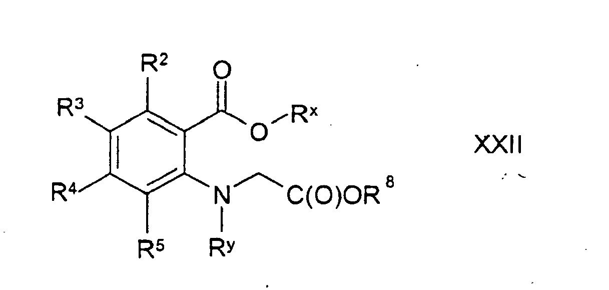 wherein R^{x} represents a C₁₋₆ alkyl group, R^{y} represents either R¹ as hereinbefore defined (as required for formation of compounds of fonnula XIV), hydrogen (as required for fonnation of compounds of formula XVII) or a nitrogen-protected derivative thereof, and R², R³, R⁴, R⁵ and R⁸ are as hereinbefore defined and, under standard cyclisation conditions known to those skilled in the art.

Compounds of formulae VIII and XII may be prepared by reaction of a compound of formula XXIII, 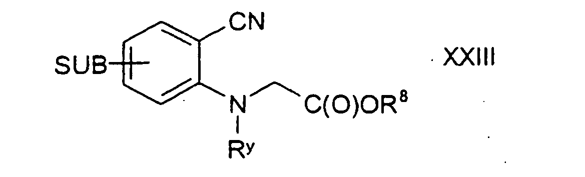 wherein SUB, R⁸ and R^{y} are as hereinbefore defined, for example under intramolecular cyclisation conditions known to those skilled in the art.

Compounds of formula XIX may be prepared by:
(a) reaction of a compound of formula XXIV, 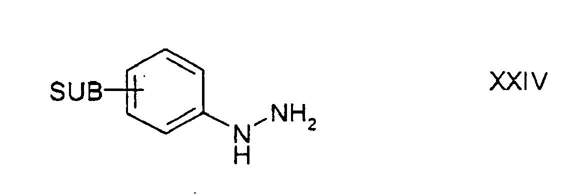 wherein SUB is as hereinbefore defined with a compound of formula XXV, 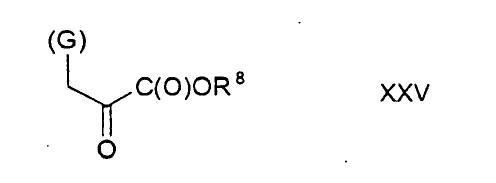 wherein (G) and R⁸ are as hereinbefore defined under condensation conditions known to the skilled person; or
(b) reaction of a compound of formula XXVI, 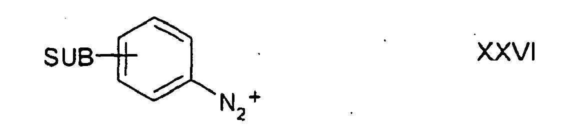 wherein SUB is as hereinbefore defined with a compound of formula XXVII, 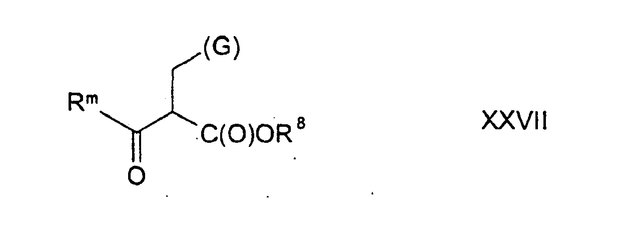 wherein R^{m} represents OH, O-C₁₋₆ alkyl-, C₁₋₆ alkyl and (G) and R⁸ are as hereinbefore defined, for example under Japp-Klingemann conditions known to the skilled person.

Compounds of formulae XX, XXI, XXII, XXIII, XXIV, XXV, XXVI and XXVII are either commercially available, are known in the literature, or may be obtained either by analogy with the processes described herein, or by conventional synthetic procedures, in accordance with standard techniques, from available starting materials using appropriate reagents and reaction conditions. In this respect, the skilled person may refer to *inter alia* "Comprehensive Organic Synthesis" by B. M. Trost and I. Fleming, Pergamon Press, 1991.

The substituents R¹, R², R³, R⁴, R⁵, R⁶, R⁷ and R⁸ in final compounds of the invention or relevant intermediates may be modified one or more times, after or during the processes described above by way of methods that are well known to those skilled in the art. Examples of such methods include substitutions, reductions, oxidations, alkylations, hydrolyses, esterifications, and etherifications. The precursor groups can be changed to a different such group, or to the groups defined in formula I**,** at any time during the reaction sequence. For example, in cases where R⁸ does not initially represent hydrogen (so providing an ester functional group), the skilled person will appreciate that at any stage during the synthesis (e.g. the final step), the relevant substituent may be hydrolysed to form a carboxylic acid functional group (in which case R⁸ will be hydrogen). In this respect, the skilled person may also refer to "Comprehensive Organic Functional Group Transformations" by A. R. Katritzky, O. Meth-Cohn and C. W. Rees, Pergamon Press, 1995.

Compounds of the invention may be isolated from their reaction mixtures using conventional techniques.

It will be appreciated by those skilled in the art that, in the processes described above and hereinafter, the functional groups of intermediate compounds may need to be protected by protecting groups.

The protection and deprotection of functional groups may take place before or after a reaction in the above-mentioned schemes.

Protecting groups may be removed in accordance with techniques that are well known to those skilled in the art and as described hereinafter. For example, protected compounds/intermediates described herein may be converted chemically to unprotected compounds using standard deprotection techniques.

The type of chemistry involved will dictate the need, and type, of protecting groups as well as the sequence for accomplishing the synthesis.

The use of protecting groups is fully described in "Protective Groups in Organic Chemisty", edited by J W F McOmie, Plenum Press (1973), and "Protective Groups in Organic Synthesis", 3rd edition, T.W. Greene & P.G.M. Wutz, Wiley-Interscience (1999).

### Medical and Pharmaceutical Uses

Compounds of the invention are indicated as pharmaceuticals. According to a further aspect of the invention there is provided a compound of the invention for use as a pharmaceutical.

Compounds of the invention are particularly useful because they may inhibit (for example selectively) the activity of prostaglandin E synthases (and particularly microsomal prostaglandin E synthase-1 (mPGES-1)), i.e. they prevent the action of mPGES-1 or a complex of which the mPGES-1 enzyme forms a part, and/or may elicit a mPGES-1 modulating effect, for example as may be demonstrated in the test described below. Compounds of the invention may thus be useful in the treatment of those conditions in which inhibition of a PGES, and particularly mPGES-1, is required.

Compounds of the invention may inhibit the activity of leukotriene C₄ (LTC₄) synthase, for example as may be shown in a test such as that described in Eur. J. Biochem., 209, 725-734 (1992), and may thus be useful in the treatment of those conditions in which inhibition of LTC₄ synthase is required. Compounds of the invention may also inhibit the activity of 5-lipoxygenase-activating protein (FLAP), for example as may be shown in a test such as that described in Mol. Pharmacol., 41, 873-879 (1992).

Compounds of the invention are thus expected to be useful in the treatment of inflammation.

The tenn "inflammation" will be understood by those skilled in the art to include any condition characterised by a localised or a systemic protective response, which may be elicited by physical trauma, infection, chronic diseases, such as those mentioned hereinbefore, and/or chemical and/or physiological reactions to external stimuli (e.g. as part of an allergic response). Any such response, which may serve to destroy, dilute or sequester both the injurious agent and the injured tissue, may be manifest by, for example, heat, swelling, pain, redness, dilation of blood vessels and/or increased blood flow, invasion of the affected area by white blood cells, loss of function and/or any other symptoms known to be associated with inflammatory conditions.

The term "inflammation" will thus also be understood to include any inflammatory disease, disorder or condition *per se,* any condition that has an inflammatory component associated with it, and/or any condition characterised by inflammation as a symptom, including *inter alia* acute, chronic, ulcerative, specific, allergic and necrotic inflammation, and other forms of inflammation known to those skilled in the art. The term thus also includes, for the purposes of this invention, inflammatory pain, pain generally and/or fever.

Accordingly, compounds of the invention may be useful in the treatment of inflammatory bowel disease, irritable bowel syndrome, migraine, headache, low back pain, fibromyalgia, myofascial disorders, viral infections (*e.g*. hepatitis C and, particularly, influenza, common cold, herpes zoster, and AIDS), bacterial infections, fungal infections, dysmenorrhea, burns, surgical or dental procedures, malignancies (*e.g*. breast cancer, colon cancer, and prostate cancer), atherosclerosis, gout, arthritis, osteoarthritis, juvenile arthritis, rheumatoid arthritis, fever (e.g. rheumatic fever), ankylosing spondylitis, systemic lupus erythematosus, vasculitis, pancreatitis, nephritis, bursitis, conjunctivitis, iritis, scleritis, uveitis, wound healing, dermatitis, eczema, psoriasis, stroke, diabetes mellitus, neurodegenerative disorders such as Alzheimer's disease and multiple sclerosis, autoimmune diseases, osteoporosis, asthma, chronic obstructive pulmonary disease, pulmonary fibrosis, allergic disorders, rhinitis, ulcers, coronary heart disease, sarcoidosis and any other disease with an inflammatory component. Other diseases that may be mentioned include inflammatory pain, hyperprostaglandin E syndrome, classic Bartter syndrome, Hodgkin's disease and persistent ductus (PDA).

Compounds of the invention may also have effects that are not linked to inflammatory mechanisms, such as in the reduction of bone loss in a subject. Conditions that may be mentioned in this regard include osteoporosis, osteoarthritis, Paget's disease and/or periodontal diseases. Compounds the invention may thus also be useful in increasing bone mineral density, as well as the reduction in incidence and/or healing of fractures, in subjects.

Compounds of the invention are indicated both in the therapeutic and/or prophylactic treatment of the above-mentioned conditions.

"Patients" include mammalian (including human) patients.

The term "effective amount" refers to an amount of a compound, which confers a therapeutic effect on the treated patient. The effect may be objective (i.e. measurable by some test or marker) or subjective (i.e. the subject gives an indication of or feels an effect).

Compounds of the invention will normally be administered orally, intravenously, subcutaneously, buccally, rectally, dermally, nasally, tracheally, bronchially, sublingually, by any other parenteral route or *via* inhalation, in a pharmaceutically acceptable dosage form.

Compounds of the invention may be administered alone, but are preferably administered by way of known pharmaceutical formulations, including tablets, capsules or elixirs for oral administration, suppositories for rectal administration, sterile solutions or suspensions for parenteral or intramuscular administration, and the like.

Such formulations may be prepared in accordance with standard and/or accepted pharmaceutical practice.

According to a further aspect of the invention there is thus provided a pharmaceutical formulation including a compound of the invention, as hereinbefore defined, in admixture with a pharmaceutically acceptable adjuvant, diluent or carrier.

Compounds of the invention may also be combined with other therapeutic agents that are useful in the treatment of inflammation (e.g. NSAIDs and coxibs).

According to a further aspect of the invention, there is provided a combination product comprising:
(A) a compound of the invention, as hereinbefore defined; and
(B) another therapeutic agent that is useful in the treatment of inflammation,
wherein each of components (A) and (B) is formulated in admixture with a pharmaceutically-acceptable adjuvant, diluent or carrier.

Such combination products provide for the administration of a compound of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention, as hereinbefore defined, another therapeutic agent that is useful in the treatment of inflammation, and a pharmaceutically-acceptable adjuvant, diluent or carrier; and
(2) a kit of parts comprising components:
   (a) a pharmaceutical formulation including a compound of the invention, as hereinbefore defined, in admixture with a pharmaceutically-acceptable adjuvant, diluent or carrier; and
   (b) a pharmaceutical formulation including another therapeutic agent that is useful in the treatment of inflammation in admixture with a pharmaceutically-acceptable adjuvant, diluent or carrier,
which components (a) and (b) are each provided in a form that is suitable for administration in conjunction with the other.

Compounds of the invention may be administered at varying doses. Oral, pulmonary and topical dosages may range from between about 0.01 mg/kg of body weight per day (mg/kg/day) to about 100 mg/kg/day, preferably about 0.01 to about 10 mg/kg/day, and more preferably about 0.1 to about 5.0 mg/kg/day. For e.g. oral administration, the compositions typically contain between about 0.01 mg to about 500 mg, and preferably between about 1 mg to about 100 mg, of the active ingredient. Intravenously, the most preferred doses will range from about 0.001 to about 10 mg/kg/hour during constant rate infusion. Advantageously, compounds may be administered in a single daily dose, or the total daily dosage may be administered in divided doses of two, three or four times daily.

In any event, the physician, or the skilled person, will be able to determine the actual dosage which will be most suitable for an individual patient, which is likely to vary with the route of administration, the type and severity of the condition that is to be treated, as well as the species, age, weight, sex, renal function, hepatic function and response of the particular patient to be treated. The above-mentioned dosages are exemplary of the average case; there can, of course, be individual instances where higher or lower dosage ranges are merited, and such are within the scope of this invention.

Compounds of the invention may have the advantage that they are effective, and preferably selective, inhibitors of prostaglandin E synthases (PGES) and particularly microsomal prostaglandin E synthase-1 (mPGES-1). The compounds of the invention may reduce the formation of the specific arachidonic acid metabolite PGE₂ without reducing the formation of other COX generated arachidonic acid metabolites, and thus may not give rise to the associated side-effects mentioned hereinbefore.

Compounds of the invention may also have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or othervvise.

### Biological Test

In the assay human mPGES-1 catalyses the reaction where the substrate PGH₂ is converted to PGE₂, mPGES-1 is expressed in *E*. *coli* and the membrane fraction is dissolved in 20mM NaPi-buffer pH 8.0 and stored at -80 °C. In the assay human mPGES-1 is dissolved in 0.1 M KPi-buffer pH 7.35 with 2.5 mM glutathione. The stop solution consists of H₂O / MeCN (7/3), containing FeCl₂ (25 mM) and HCl (0.15 M). The assay is performed at room temperature in 96-well plates. Analysis of the amount of PGE₂ is performed with reversed phase HPLC (Waters 2795 equipped with a 3.9 x 150 mm C18 column). The mobile phase consists of H₂O / MeCN (7/3), containing TFA (0.056%), and absorbance is measured at 195 nm with a Waters 248.7 UV-detector.
The following is added chronologically to each well:
1. 100 µL human mPGES-1 in KPi-buffer with glutathione. Total protein concentration: 0.02 mg/mL.
2. 1 µL inhibitor in DMSO. Incubation of the plate at room temperature for 25 minutes.
3. 4 µL of a 0.25 mM PGH₂ solution. Incubation of the plate at room temperature for 60 seconds.
4. 100 µL stop solution.
   180 µL per sample is analyzed with HPLC.

### Examples

The invention is illustrated by way of the following examples, in which the following abbreviations may be employed:

| | |
|---|---|
| BINAP | 2,2'-bis(diphenylphosphmo)-1,1'-binaphtyl |
| dba | dibenzylideneacetone |
| DIBAL | diisobutylaluminium hydride |
| DMAP | 4,4-dimethylaminopyridine |
| DMF | dimethylformamide |
| DMSO | dimethylsulfoxide |
| EtOAc | ethyl acetate |
| HPLC | High Pressure Liquid Chromatography |
| MeCN | acetoi-dtrile |
| MS | mass spectrum |
| NBS | *N*-bromosuccinimide |
| NMR | nuclear magnetic resonance |
| TFA | trifluoroacetic acid |
| THF | tetrahydrofuran |
| xantphos | 9,9-dimethyl-4,5-bis(diphenylphosphino)-xanthene |

Starting materials and chemical reagents specified in the syntheses described below are commercially available from, *e.g.* Sigma-Aldrich Fine Chemicals.

### Example 1

### 3-Acetamido-1,5-bis(4-iso propoxyphenyl)-indole-2-carboxylic acid

### (a) 5-(4-Isopropoxyphenyl)indole-2-carboxylic acid ethyl ester

A mixture of 5-bromoindole-2-carboxylic acid ethyl ester (2 g, 7.5 mmol), 4-isopropoxyphenylboronic acid (2.72 g, 15 mmol), K₃PO₄ (5.52 g, 26 mmol), Pd(OAc)₂ (85 mg, 0.38 mmol), tri-o-tolylphosphine (228 mg, 0.75 mmol), EtOH (20 ml) and toluene (10 mL) was stirred under argon for 20 min at room temperature, and then heated at 100°C for 24 h. The mixture was allowed to cool, poured into NaHCO₃ (aq., sat.) and extracted with EtOAc. The combined extracts were washed with water and brine and then dried over Na₂SO₄. Concentration and purification by chromatography gave the sub-title compound (1.81 g, 98%).

### (b) 3-Bromo-5-(4-isopropoxyphenyl)indole-2-carboxylic acid ethyl ester

A solution of NBS (0.90 g, 5.1 mmol) in acetone (10 mL) was added dropwise to a stirred solution of 5-(4-isopropoxyphenyl)indole-2-carboxylic acid ethyl ester (1.5 g, 4.62 mmol; see step (a)) in acetone (35 mL) at room temperature. After 2.5 h, additional NBS (164 mg, 0.92 mnol) was added and the temperature was raised to 45 °C and the mixture was stirred at that temperature for 1.5 h. The mixture was allowed to cool, poured into Na₂S₂O₃ (aq. 10 %), and extracted with EtOAc. The combined extracts were washed with Na₂S₂O3 (aq. 10%), NaHCO₃ (aq. sat), brine and dried over Na₂SO₄. The solvents were evaporated under reduced pressure and the residue crystallised from ethanol to yield the sub-title compound (1.63 g, 88%).

### (c) 3-Bromo-1,5-bis(4-isopropoxyphenyl)indole-2-carboxilic acid ethyl ester

A mixture of 3-bromo-5-(4-isopropoxyphenyl)indole-2-carboxylic acid ethyl ester, ethyl ester (700 mg, 1.74 mmol; see step (b)), Cu(OAc)₂ (632 mg, 3.48 mmol), Et₃N (489 µL, 3.48 mmol), pyridine (284 µL, 3.48 mmol), 4-isopropoxyphenylboronic acid (626 mg, 3.48 mmol) and 3Å molecular sieves in dichloroethane was stirred vigorously at ambient temperature for 30 h. The mixture was filtered through Celite^{®}, the filter cake washed with EtOAc and the solvents concentrated. The residue was purified by chromatography to give the sub-title compound (831 mg, 89 %).

### (d) 3-Acetamido-1.5-bis(4-isopropoxyphenyl)indole-2-carboxylic acid ethyl ester

3-Bromo-1,5-bis(4-isopropoxyphenyl) indole-2-carboxylic acid ethyl ester (230 mg, 0.43 mmol) in dioxane (3 mL), followed by *N,N*'-dimethylethylenediamine (14 µL, 12 mg, 0.13 mmol) were added whilst stirring to acetamide (76 mg, 1.29 mmol), CuI (8 mng, 0.04 mmol) and K₃PO₄ (191 mg, 0.90 mmol) in a pressure tube under argon at room temperature. The septum inlet was replaced with a teflon screw-cap and the mixture was heated at 100°C for 16 hours. The mixture was subsequently cooled and then filtered through Celite^{®} and the filter cake washed with EtOAc. The combined filtrates were concentrated and purified by chromatography to afford the title compound (149 mg, 67 %).

### (e) 3-Acetamido-1,5-bis(4-isopropoxyphenyl)indole-2-carboxylic acid

A mixture of 3-acetamido-1,5-bis(4-isopropoxyphenyl)indole-2-carboxylic acid ethyl ester (129 mg, 0.250 mmol; see step (d)), aqueous NaOH (1M, 2 mL) and MeCN (2 mL) was heated under microwave irradiation at 120 °C for 1.5 h, allowed to cool, then acidified with HCl (aq., 1M) to pH 2 and extracted with EtOAc. The combined extracts were washed with water, brine and dried over Na₂SO₄. Concentration, purification by chromatograpy, and recrystallisation from ethyl acetate and hexanes gave the title compound.
200 MHz ¹H-NMR (DMSO-d₆, ppm) δ 13.1-12.7 (1H, br s), 9.78 (1H, s), 7.85-7.80 (1H, m), 7.58-7.48 (3H, m), 7.29-7.20 (2H, m), 7.08-6.96 (5H, m), 4.68 (1H, septet, J=6.0 Hz), 4.64 (1H, septet, J=6.0 Hz), 2.15 (3H, s), 1.33 (6H, d, J=6.0 Hz), 1.28 (6H, d, J=6.0 Hz).

### Example 2

### 3-(4-(Dimethylamino)butanamido)-1,5-bis(4-isopropoxyphenyl)indole-2-carboxylic acid

The title compound was prepared in accordance with Example 1 using 4-(dimethylamino)butyric acid amide in Example 1 (d) instead of acetamide. 200 MHz ¹H-NMR (DMSO-d₆/CF₃COOD, ppm) δ 9.9 (1H, s), 9.6-9.4 (1H, br s), 7.81 (1H, s), 7.59-7.49 (3H, m), 7.29-7.20 (2H, m), 7.10-6.96 (5H, m), 4.68 (1H, septet, J=6.0 Hz), 4.65 (1H, septet, J=6.0 Hz), 3.23-3.07 (2H, m), 2.82 (3H, s), 2.80 (3H, s), 2.62-2.52 (2H, m), 1.33 (6H, d, J=6.0-Hz), 1.28 (6H, d, J=6.0 Hz).

### Example 3

### 1,5-Bis(4-ispropoxyphenyl)-3-(methylsulfonamido)indole-2-carboxylic acid

### (a) 1,5-Bis(4-isopropoxyphenyl)-3-(methylsulfonamido)indole-2-carboxylic acid ethyl ester

A mixture of 3-bromo-1,5-bis(4-isopropoxyphenyl)indole-2-carboxylic acid ethyl ester (200 mg, 0.37 mmol; see Example 1 (c)), methanesulfonamide (71 mg, 0.74 mmol), Pd₂(dba)₃ (17 mg, 0.019 mmol), xantphos (33 mg, 0.057 mmol), Cs₂CO₃ (181 mg, 0.56 mmol) and dioxane (3 mL) was heated under argon at 110 °C for 20 h, and then allowed to cool to room temperature. Water was added and the mixture was extracted with EtOAc. The combined extracts were washed with brine, dried over Na₂SO₄, and concentrated. Purification by chromatography yielded the sub-title compound (120 mg, 59%).

### (b) 1,5-Bis(4-isopropoxyphenyl)-3-(methylsulfonamido)indole-2-carboxylic acid

The title compound was prepared by hydrolysis of 1,5-bis(4-isopropoxyphenyl)-3-(methylsulfonamido)indole-2-carboxyic acid ethyl ester (see step (a) above) in accordance with the procedure described in Example 1 (e).
200 MHz ¹H-NMR (DMSO-d₆, ppm) δ 13.5-13.0 (1H, br s), 9.21 (1H, s), 7.97 (1H, d, J=1.2 Hz), 7.61-7.47 (3H, m), 7.36-7.26 (2H, m), 7.11-6.96 (5H, m), 4.69 (1H, septet, J=6.0 Hz), 4.65 (1H, septet, J=6.0 Hz), 3.02 (3H, s), 1.33 (6H, d, J=6.0 Hz), 1.29 (6H, d, J=6.0 Hz).

### Example 4

### 1,5-Bis(4-isopropoxyphenyl)-3-(N-(3-phenylpropyl)-4-(trifluoromethoxy)-benzamido)indole-2-carboxylic acid

### (a) 1,5-Bis(4-isopropoxyphenyl)-3-(3-phenylpropylamino)indole-2-carboxylic acid ethyl ester

A mixture of 3-bromo-1,5-bis(4-isopropoxyphenyl)indole-2-carboxylic acid ethyl ester (210 mg, 0.39 mmol; see Example 1 (c)), 3-phenyl-1-propylamine (67 µL, 63 mg, 0.47 mmol), Pd₂(dba)₃ (12.5 mg, 0.014 mmol), BINAP (37 mg, 0.052 mmol) and Cs₂CO₃ (178 mg, 0.54 mmol) in toluene (2 mL) was heated under argon at 120 °C for 16 h. Water was added and the mixture was extracted with EtOAc. The combined extracts were washed with brine, dried over Na₂SO₄, and concentrated. Purification by chromatography yielded the title compound.

### (b) 1,5-Bis(4-isopropoxyphenyl)-3-(N-(3-phenylpropyl)-4-(trifluoromethoxy)benzamido)indole-2-carboxyic acid ethyl ester

4-(Trifluoromethoxy)benzoyl chloride (61 µL, 87 mg, 0.385 mmol) was added to a solution of 1,5-bis(4-isopropoxyphenyl)-3-(3-phenylpropylamino)indole-2-carboxylic acid ethyl ester (200 mg, 0.34 mmol; see step (a)) in toluene (3 mL) at room temperature. The reaction mixture was heated under argon at 80°C for 50 min and then allowed to cool. NaHCO₃ (aq. sat., 30 mL) was added and the mixture stirred for 40 min, after which it was extracted with EtOAc. The combined extracts were washed with brine, dried over Na₂SO₄, and concentrated. Purification by chromatography yielded the sub-title compound.

### (c) 1,5-Bis(4-isopropoxyphenyl-3-(N-(3-phenylpropyl)-4-(trifluoromethoxy)benzamido)indole-2-carboxylic acid

The title compound was prepared by hydrolysis of 1,5-bis(4-isopropoxypbenyl)-3-(*N*-(3-phenylpropyl)-4-(trifluoromethoxy)benzamido)-indole-2-carboxylic acid ethyl ester (see step (b)) in accordance with the procedure described in Example 1(e).
200 MHz ¹H-NMR (DMSO-d₆, ppm) δ 7.77-7.31 (6H, m), 7.26-6.85 (14H, m), 4.73-4.53 (2H, m), 4.14-3.69 (2H, m), 2.71-2.54 (2H, m), 1.99-1.77 (2H, m), 1.36-1.20 (12H, m).

### Example 5

### 1,5-Bis(4-isopropoxphenyl)-3-(N-(pyrid-3-ylmethyl)acetamido)indole-2-carboxylic acid

The title compound was prepared in accordance with the procedures described in Example 4 using pyrid-3-ylmethanamine and acetyl chloride instead of 3-phenyl-1-propylamine and 4-(trifluoromethoxy)benzoyl chloride.
200 MHz ¹H-NMR (DMSO-d₆, ppm) δ 8.53-8.36 (2H, m), 7.73 (1H, d, J=7.6 Hz), 7.42-7.20 (6H, m), 7.10-6.88 (6H, m), 5.36 (1H, d, J=14.1 Hz), 4.65 (1H, septet, J=6.1 Hz), 4.62 (1H, septet, J=6.0 Hz), 4.50 (1H, d, J=14.1 Hz), 1.85 (3H, s), 1.31 (6H, d, J=6.1 Hz) 1.26 (6H, d, J=6.0 Hz).

### Example 6

### 3-(4-Chlorobenzamido)-1-(4-isopropoxyphenyl)-5-(4-(trifluoromethyl)-phenyl)indole-2-carboxylic acid

### (a) Ethyl 3-iodo-5-(4-(trifluoromethyl)phenyl)indole-2-carboxylate

The reaction was performed with the exclusion of light. NaI (2.04 g, 13.6 mmol) was added portion-wise whilst stirring to *N*-chlorosuccinimide (1.83 g, 13.6 mmol) in acetone (125 mL), followed by 5-(4-(trifluoromethyl)phenyl)indole-2-carboxylic acid ethyl ester (3.8 g, 11.4 mmol; which compound was prepared in accordance with the procedure described in Example 1 (a) using 4-trifluoromethylphenyl boronic acid instead of 4-isopropoxyphenyl boronic acid), in acetone (60 mL) at room temperature. After 2 h the mixture was poured into Na₂S₂O₃ (aq. 10 %) and extracted with EtOAc. The combined extracts were washed with NaHCO₃ (sat. aq.), brine, and dried over Na₂SO₄. The solvents were removed under reduced pressure and the residue triturated with petroleum ether to yield the title compound (4.9 g 94%) that was used in the next step without further purification.

### (b) 3-(4-Chlorobenzamido)-1-(4-isopropoxyphenyl)-5-(4-(trifluoromethyl)-phenyl)indole-2-carboxylic acid

The title compound was prepared in accordance with the procedures described in Example 1 (c) to 1 (e), using 4-chlorobenzamide instead of acetamide.
200 MHz ¹H-NMR (DMSO-d₆, ppm) δ 11.2-10.8 (1H, br s), 8.27 (1H, s), 8.13-8.03 (2H, m), 7.93-7.73 (4H, m), 7.68-7.58 (3H, m), 7.31-7.21 (2H, m), 7.11 (1H, d, J=9.0 Hz), 7.09-6.99 (2H, m), 4.66 (1H, septet, J=6.1 Hz), 1.32 (6H, d, J=6.0 Hz).

### Example 7

### 1-(4-Isopropoxyphenyl)-3-(4-morpholino)-5-(4-(trifluoromethyl)phenyl)-indole-2-carboxylic acid

### (a) 5-(4-(Trifluoromethyl)phenyl)indole-2-carboxylic acid ethyl ester

The sub-title compound was prepared in accordance with the procedure described in Example 1 (a) using 5-bromoindole-2-carboxylic acid ethyl ester and 4-trifluoromethylphenylboronic acid instead of 4-isopropoxyphenylboronic acid.

### (b) 3-Bromo-1-(4-isopropoxyphenyl)-5-(4-(trifluoromethyl)phenyl)indole-2-carboxylic acid ethyl ester

The sub-title compound was prepared in accordance with the procedure described in Example 1(b) and 1(c) from 5-(4-(trifluoromethyl)phenyl)indole-2-carboxylic acid ethyl ester (see step (a)), NBS and 4-isopropoxyphenylboronic acid.

### (c) 1-(4-Isopropoxyphenyl)-3-(4-morpholino)-5-(4-(trifluoromethyl)-phenyl)indole-2-carboxylic acid ethyl ester

Morpholine (34.5 µL, 0.4 mmol), followed by anhydrous toluene (10 mL) was added under argon to a mixture of Pd₂(dba)₃ (6 mg, 0.0066 mmol), BINAP (6.12 mg, 0.0099 mmol), Cs₂CO₃ (150 mg, 0.46 mmol) and 3-bromo-1-(4-isopropoxyphenyl)-5-(4-(trifluoromethyl)phenyl)indole-2-carboxylic acid ethyl ester (180 mg, 0.33 mmol; see step (b)). The mixture was stirred at 100°C for 24 h, after which an additional portion of BINAP (2.1 mg, 0.0033 mmol) was added. The heating was continued for 24 h after which additional portions of Pd₂(dba)₃ (3.02 mg, 0.0033 mmol) and BINAP (3.06 mg, 0.005 mmol) were added. The mixture was heated for a further 12 h, allowed to cool, diluted with Et₂O, and filtered through Celite^{®}. The filtrate was concentrated and the residue purified by chromatography to afford the sub-title compound (116 mg, 64%).

### (d) 1-(4-Isopropoxyphenyl)-3-(4-morpholino)-5-(4-(trifluoromethyl)-phenyl)indole-2-carboxylic acid

The title compound was prepared by hydrolysis of 1-(4-isopropoxyphenyl)-3-(4-morpholino)-5-(4-(trifluoromethyl)phenyl)indole-2-carboxylic acid ethyl ester (see step (c)) in accordance with the procedure described in Example 1 (e).
200 MHz ¹H-NMR (DMSO-d₆, ppm) δ 8.27 (1H, d, J=1.4 Hz), 8.02-7.94 (2H, m), 7.85-7.76 (2H, m), 7.67 (1H, dd, J=1.4, 8.8 Hz), 7.31-7.22 (2H, m), 7.16 (1H, d, J=8.8 Hz), 7.07-6.99 (2H, m), 4.68 (1H, septet, J=6.0 Hz), 3.91-3.76 (4H, m), 3.50-3.38 (4H, m), 1.32 (6H, d, J=6.0).

### Example 8

### 3-(Acetylmethylamino)-5-(4-tert-butylphenyl)-1-(4-isopropoxyphenyl)-1H-indole-2-carboxylic acid

### (a) 5-Bromo-3-nitro-1H-indole-2-carboxylic acid ethylester

Cu(NO₃)₂ was added to acetic anhydride (10 mL) at -5°C (whilst stirring the mixture). After 10 min, a solution of 5-bromo,- 1H-indole-2-carboxylic acid ethyl ester (2.0 g, 7.46 mmol) in acetic anhydride (25 mL) was added portion-wise. The mixture was stirred for 2 h at -5°C, solid was removed by filtration and washed with acetic anhydride. The filtrate was poured into ice-water (150 mL) and stirred for 5 h. The precipitate was filtered, washed with water and dried to afford the sub-title compound (2.2 g, 94%).

### (b) 5-Bromo-1-(4-isopropoxyphenyl)-3-nitro-1H-indole-2-carboxylic acid ethyl ester

The sub-title compound was prepared from 5-bromo-3-nitro-1H-indole-2-carboxylic acid ethyl ester (see step (a) above) in accordance with the procedure described in Example I (c).

### (c) 5-(4-tert-Butylphenyl)-1-(4-isopropoxyphenyl)-3-nitro-1H-indole-2-carboxylic acid ethyl ester

The sub-title compound was prepared in accordance with the procedure described in Example 1(a) using 5-bromo-1-(4-isopropoxyphenyl)-3-nitro-1H-indole-2-carboxylic acid ethyl ester (see step (b) above) and 4-*tert-*butylphenylboronic acid instead of 4-isopropoxyphenylboronic acid.

### (d) 3-Amino-5-(4-tert-butylphenyl)-1-(4-isopropoxyphenyl)-1H-indole-2-carboxylic acid ethyl ester

Pd-C (550 mg of 10%) was added to the solution of 5-(4-*tert*-butylphenyl)-1-(4-isopropoxyphenyl)-3-nitro-1H-indole-2-carboxylic acid ethyl ester (1.1 g, 2.20 mmol; see step (c) above) in EtOAc (50 mL) and the mixture was stirred for 10 h under hydrogen (1 atm). After filtration through Celite^{®}, the filtrate was concentrated and the residue purified by chromatography to afford the sub-title compound (760 mg, 73%).

### (e) 5-(4-tert-Butylphenyl)-1-(4-isopropoxyhenyl)-3-methylamino-1H-indole-2-carboxylic acid ethyl ester

A solution of 3-amino-5-(4-*tert*-butylphenyl)-1-(4-isopropoxyphenyl)-1H-indole-2-carboxylic acid ethyl ester (300 mg, 0.64 mmol; see step (d) above) in DMF (3 mL) was added to a stirred suspension of NaH (23 mg, 0.70 mmol) in DMF (1 mL) at 0°C. After stirring at 0°C for 30 min, a solution of methyl iodide (60 µL, 0.96 mmol) in DMF (1mL) was added portion-wise. The reaction was left to stir at room temperature for 14 h, then poured into water and extracted with EtOAc. The combined organic extracts were washed with water, brine and dried (Na₂SO₄). Removal of the solvent and purification by chromatography afforded the sub-title compound (200 mg, 64%).

### (f) 3-(Acetylmethylamino)-5-(4-tert-butylphenyl)-1-(4-isopropoxyphenyl)-1H-indole-2-carboxylic acid ethyl ester

A mixture of 5-(4-*tert*-butylphenyl)-1-(4-isopropoxyphenyl)-3-methylamino-1H-indole-2-carboxylic acid ethyl ester (200 mg, 0.41 mmol; see step (d) above), acetyl chloride (59 µL. 0.82 mmol), triethylamine (115 µL, 0.82 mmol) and dry MeCN (5 mL) was stirred at room temperature under argon for 1.5 h. The mixture was poured into HCl (1N) and extracted with EtOAc. The combined organic extracts were washed with water, brine and dried (Na₂SO₄). Removal of the solvent and purification by chromatography yielded the sub-title compound (87 mg, 40%).

### (g) 3-(Acetylmethylamino)-5-(4-tert-butylphenyl)-1-(4-isopropoxyphenyl)-1H-indole-2-carboxylic acid

The title compound was prepared by hydrolysis of 3-(acetylmethylamino)-5-(4-*tert*-butylphenyl)-1-(4-isopropoxyphenyl)-1H-indole-2-carboxylic acid ethyl ester (see step (f) above) in accordance with the procedure described in Example 1(e).
200 MHz ¹H-NMR (acetone-d₆, ppm) δ 7.91-7.89 (1H, m) 7.72-7.64 (3H, m) 7.55-7.48 (2H, m) 7.41-7.32 (2H, m) 7.22 (1H, dd, J=8.8, 0.7 Hz) 7.13-7.05 (2H, m) 4.73 (1H, septet, J=6.0 Hz) 3.33 (3H, s) 1.89 (3H, s) 1.38 (6H, d, J=6.0 Hz) 1.36 (9H, s).

### Example 9

### 1-(4-Cyclopentoxyphenyl)-3-(4-dimethylaminobutyrylamino)-5-(5-trifluoromethylpyrid-2-yl)-1H-indole-2-carboxylic acid

### (a) 5-(4,4,5,5-Tetramethyl[1,3,2]dioxaborolan-2-yl)-1H-indole-2-carboxylic acid ethyl ester

Pd₂(dba)₃ (275 mg, 0.30 mmol) and tricyclohexylphosphine (504 mg, 1.80 mmol) in dioxane (30 mL) were added under argon to a stirred mixture of 5-bromo-1H-indole-2-carboxylic acid ethyl ester (6.0 g, 22.4 mmol), KOAc (3.3 g, 33.6 mmol), bis(pinacolato)diboron (6.3 g, 24.6 mmol) and dioxane (20 mL) at. 80°C. The resulting mixture was stirred at 80°C for 3 h, cooled to room temperature and filtered through a Celite® pad. The filter cake was washed with EtOAc and the combined filtrates were concentrated and purified by chromatography to yield the sub-title compound (6.8 g, 97%).

### (b) 5-(5-Trifluoromethylpyrid-2-yl)-1H-indole-2-carboxylic acid ethyl ester

A stirred mixture of 5-(4,4,5,5-tetramethyl[1,3,2]dioxaborolan-2-yl)-1H-indole-2-carboxylic acid ethyl ester (3.00 g, 9.52 mmol; see step (a) above), 2-bromo-5-(trifluoromethyl)pyridine (3.23 g, 14.28 mmol), sodium carbonate (2M, 14.30 mL, 28.56 mmol), Pd(PPh₃)₄ (540 mg, 0.50 mmol), EtOH (10 mL) and toluene (40 mL) were heated at 80°C for 24 h. The mixture was cooled to room temperature, poured into water and extracted with EtOAc. The combined extracts were washed with water, brine and dried (Na₂SO₄). Solvent removal and purification by chromatography gave the sub-title compound (3.0 g, 94%).

### (c) 3-Iodo-5-(5-trifluoromethylpyrid-2-yl)-1H-indole-2-carboxylic acid ethyl ester

The sub-title compound was prepared in accordance with the procedure described in Example 6(a) using 5-(5-trifluoromethylpyrid-2-yl)-1H-indole-2-carboxylic acid ethyl ester (see step (b) above).

### (d) 1-(4-Cyclopentoxyphenyl)-3-iodo-5-(5-trifluoromethylpyrid-2-yl)-1H-indole-2-carboxylic acid ethyl ester

The sub-title compound was prepared in accordance with the procedure described in Example 1(c) using 3-iodo-5-(5-trifluoromethylpyrid-2-yl)-1H-indole-2-carboxylic acid ethyl ester (see step (c) above) and 4-cyclopentoxyphenylboronic acid instead of 4-isopropoxyphenylboronic acid.

### (e) 1-(4-Cyclopentoxyphenyl)-3-(4-dimethylaminobutyrylamino)-5-(5-trifluoromethylpyrrid-2-yl)-1H-indole-2-carboxylic acid ethyl ester

The sub-title compound was prepared in accordance with the procedure described in Example 1(d) using 1-(4-cyclopentoxyphenyl)-3-iodo-5-(5-trifluoromethylpyrid-2-yl)-1H-indole-2-carboxylic acid ethyl ester (see step (d) above) and 4-dimethylaminobutyramide instead of acetamide.

### (f) 1-(4-Cyclopentoxyphenyl)-3-(4-dymethylaminobutyrylamino)-5-(5-trifluoromethylpyrid-2-yl)-1H-indole-2-carboxylic acid

The title compound was prepared by hydrolysis of 1-(4-cyclopentoxyphenyl)-3-(4-dimethylaminobutyrylamino)-5-(5-trifluoromethylpyrid-2-yl)-1H-indole-2-carboxylic acid ethyl ester (see step (e)) in accordance with the procedure described in Example 1(e).
200 MHz ¹H-NMR (DMSO-d₆, ppm) δ 11.5-10.5 (1H, br s) 9.03-8.97 (1H, m) 8.91-8.86 (1H, m) 8.28-8.19 (1H, m) 8.08 (1H, d, J=8.2 Hz) 8.01-7.93 (1H, m) 7.23-7.12 (2H, m) 7.04 (1H, d, J=9.0 Hz) 7.01-6.92 (2H, m) 4.90-4.78 (1H, m) 3.02-2.90 (2H, m) 2.91 (6H, s) 2.58-2.50 (2H, m, overlapped with DMSO) 2.09-1.53 (10H, m).

### Example 10

### 1-(4-Cyclopentoxyphenyl)-3-(2-oxopyrrolidin-1-yl)-5-(5-trifluoromethyl-pyrid-2-yl)-1H-indole-2-carboxylic acid

### (a) 1-(4-Cyclopentoxyphenyl)-3-(2-oxopyrrolidin-1-yl)-5-(5-trifluoro-methylpyrid-2-yl)-1H-indole-2-carboxylic acid ethyl ester

The sub-title compound was prepared in accordance with the procedure described in Example 1(d) using 1-(4-cyclopentoxyphenyl)-3-iodo-5-(5-trifluoromethylpyrid-2-yl)-1H-indole-2-carboxylic acid ethyl ester (see Example 9(d)) and pyrrolidin-2-one instead of acetamide.

### (b) 1-(4-Cyclopentoxyphenyl)-3-(2-oxopyrrolidin-1-yl)-5-(5-trifluoro-methylpyrid-2-y)-1H-indole-2-carboxylic acid

The title compound was prepared by hydrolysis of 1-(4-cyclopentoxyphenyl)-3-(2-oxopyrrolidin-1-yl)-5-(5-trifluoromethylpyrid-2-yl)-1H-indole-2-carboxylic acid ethyl ester (see step (a) above) in accordance with the procedure described in Example 1 (e).
200 MHz ¹H-NMR (DMSO-d₆, ppm) δ 13.13 (1H, s) 9.05-9.01 (1H, m) 8.42-8.39 (1H, m) 8.33-8.22 (2H, m) 8.16 (1H, dd, J=8.9,1.6 Hz) 7.36-7.27 (2H, m) 7.19 (1H, d, J=8.9 Hz) 7.10-7.01 (2H, m) 4.95-4.85 (1H, m) 3.95-3.83 (2H, m) 2.50-2.43 (2H, m, overlapped with DMSO) 2.33-2.14 (2H, m) 2.07-1.87 (2H, m) 1.87-1.55 (6H, m).

### Example 11

### 3-Acetylamino-1-(4-isopropoxyphenyl)-5-(5-trifluoromethylpyridin-2-yl)-1H-indole-2-carboxylic acid

### (a) 3-Iodo-1-(4-isopropoxyphenyl)-5-(5-trifluoromethylpyridin-2-yl)-1H-indole-2-carboxylic acid ethylester

The sub-title compound was prepared in accordance with the procedure described in Example 1 (c) using 3-iodo-5-(5-trifluoromethylpyridin-2-yl)-1H-indole-2-carboxylic acid ethyl ester (see Example 9 (c)) and 4-isopropoxyphenylboronic acid.

### (b) 3-Acetylamino-1-(4-isopropoxyphenyl)-5-(5-trifluoromethylpyridin-2-yl)-1H-indole-2-carboxylic acid ethyl ester

The sub-title compound was prepared in accordance with the procedure described in Example 1 (d) using 3-iodo-1-(4-isopropoxyphenyl)-5-(5-trifluoromethylpyridin-2-yl)-1H-indole-2-carboxylic acid ethyl ester (see step (a) above) and acetamide.

### (c) 3-Acetylamino-1-(4-isopropoxyphenyl)-5-(5-trifluoromethylpyridin-2-yl)-1H-indole-2-carboxylic acid

The title compound was prepared by hydrolysis of 3-acetylamino-1-(4-isopropoxyphenyl)-5-(5-trifluoromethylpyridin-2-yl)-1H-indole-2-carboxylic acid ethyl ester (see step (b) above) in accordance with the procedure described in Example 1(e).
200 MHz ¹H-NMR (DMSO-d_{6,} ppm) δ 13.1-13.0 (1H, br s) 9.86 (1H, s) 9.05-9.00 (1H, m) 8.51 (1H, d, J=1.4 Hz) 8.30-8.23 (1H, m) 8.17 (1H, d, J=8.6 Hz) 8.10 (1H, dd, J=8.8, 1.4 Hz) 7.33-7.23 (2H, m) 7.13(1H, d, J=8.8 Hz) 7.09-7.02 (2H, m) 4.69 (1H, septet, J=6.0 Hz) 2.17 (3H, s) 1.33 (6H, d, J=6.0 Hz).

### Example 12

### 3-(Acetylmethylamino-1-(4-isopropoxyphenyl)-5-(5-trifluoromethyl-pyridin-2-yl)-1H-indole-2-carboxylic acid

### (a) 3-(Acetylmethylamino)-1-(4-isopropoxyphenyl)-5-(5-trifluoromethyl-pyridin-2-yl)-1H-indole-2-carboxylic acid ethyl ester

A solution of 3-acetylamino-1-(4-isopropoxyphenyl)-5-(5-trifluoromethyl-pyridin-2-yl)-1H-indole-2-carboxylic acid ethyl ester (196 mg, 0.37 mmol; see Example 11 (b)) in DMF (4 mL) was added to a stirred suspension of NaH (13 mg, 0.41 mmol) in DMF (2 mL) at 0°C. After stirring at 0°C for 30 min, a solution of methyl iodide (49 µL, 0.78 mmol) in DMF (2 mL) was added portion-wise. The reaction was left to stir at room temperature for 14 h, then poured into water and extracted with EtOAc. The combined extracts were washed with water, brine and dried over Na₂SO₄. The organic phase was concentrated and the product purified by chromatography to give the sub-title compound (169 mg, 84%).

### (b) 3-(Acetylmethylamino)-1-(4-isopropoxyphenyl)-5-(5-trifluoromethyl-pyridin-2-yl)-1H-indole-2-carboxylic acid

The title compound was prepared by hydrolysis of 3-(acetylmethylamino)-1-(4-isopropoxyphenyl)-5-(5-trifluoromethylpyridin-2-yl)-1H-indole-2-carboxylic acid ethyl ester (see step (a) above) in accordance with the procedure described in Example 1 (e).
200 MHz ¹H-NMR (DMSO-d₆, ppm) δ 13.6.12.9 (1H, br s) 9.06-9.00 (1H, m) 8.48-8.44 (1H, m) 8.35 (1H, d, J=8.6 Hz) 8.29-8.21 (1H, m) 8.20 (1H, dd, J=8.8, 1.4 Hz) 7.40-7.31 (2H, m) 7.22 (1H, d, J=8.8 Hz) 7.11-7.02 (2H, m) 4.71 (1H, septet, J=6.0 Hz) 3.24 (3H, s) 1.82 (3H, s) 1.34 (6H, d, J=6.0 Hz).

### Example 13

1-(4-Isopropoxyphenyl)-3-nicotinamide-5-(5-trifluoromethylpyridin-2-yl)-1H-indole-2-carboxylic acid was prepared in accordance with the procedures described herein.

### Example 14

Title compounds of the examples were tested in the biological test described above and were found to exhibit 50% inhibition of mPGES-1 at a concentration of 10 µM or below. For example, for the following compounds of the examples, 50% inhibition was observed at:

| | |
|---|---|
| Example 1: | 3100 nM |
| Example 3: | 1700 nM |
| Example 7: | 730 nM |
| Example 12: | 5300 nM |
| Example 13: | 190 nM |

## Claims

1. A compound of formula I, 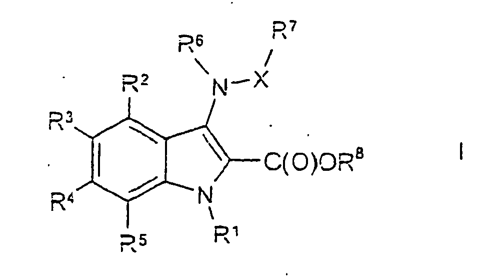 wherein
X represents a single bond, -C(O)- or -S(O)₂-;
R¹ represents an aryl group or a heteroaryl group, both of which groups are optionally substituted by one or more substituents selected from A;
one of the groups R², R³, R⁴ and R⁵ represents an aryl group or a heteroaryl group (both of which are optionally substituted by one or more substituents selected from A) and:
a) the other groups are independently selected from hydrogen, G¹, an aryl group, a heteroaryl group (which latter two groups are optionally substituted by one or more substituents selected from A), C₁₋₈alkyl and a heterocycloalkyl group (which latter two groups are optionally substituted by one or more substituents selected from G¹ and/or Z¹); and/or
b) any two other groups which are adjacent to each other are optionally linked to form, along with two atoms of the essential benzene ring in the compound of formula I, a 3- to 8-membered ring, optionally containing 1 to 3 heteroatom and/or 1 to 3 double bonds, which ring is itself optionally substituted by out or more substituents selected from halo, -R⁸, -OR⁸ and =O;
R⁶, R⁷ and R⁸ independently represent on each occasion when used above:
I) hydrogen;
II) an aryl group or a heteroaryl group, both of which are optionally substituted by one or more substituents selected from B;
III) C₁₋₈ alkyl or a heterocycloalkyl group, both of which are optionally substituted by one or more substituents selected from G¹ and/or Z¹; or
R⁶ and R⁷ may be linked together to form along with the N atom and X group to which R⁶ and R⁷ are respectively attached, a 3- to 8-membered ring, optionally containing 1 to 3 heteroatoms and/or 1 to 3 double bonds, which ring is optionally substituted by one or more substituents selected from G¹ and/or Z¹;
A represents, on each occasion when mentioned above:
I) an aryl group or a heteroaryl group, both of which are optionally substituted by one or more substituents selected from B;
II) C₁₋₈ alkyl, or a heterocycloalkyl group, both of which are optionally substituted by one or more substituents selected from G¹ and/or Z¹;
III) a G¹ group; or
IV) two A substituents may be linked together to form, along with at least two (e.g. adjacent) atoms of the aryl or heteroaryl group to which the two A substituents are attached, a further 3- to 5-membered ring, which ring optionally contains 1 to 3 heteroatoms and/or 1 to 2 double bonds, and which is optionally substituted, by halo or C₁₋₈ alkyl, which latter group is optionally substituted by halo;
G¹ represents, on each occasion when mentioned above, halo, cyano, -N₃, **-**NO₂, -ONO₂ or -A¹-R⁹;
wherein A¹, represents a single bond or a spacer group selected from -C(O)A²-, -S(O)ₙA³-, -N(R¹⁰)A⁴- or -OA⁵-, in which:
A² and A³ independently represent a single bond, -O-, -N(R¹⁰)- or -C(O)-;
A⁴ and A⁵ independently represent a single bond, -C(O)-, -C(O)N(R¹⁰)-, -C(O)O-, -S(O)ₙ- or -S(O)ₙN(R¹⁰)-;
Z¹ represents, on each occasion when mentioned above, =O, =S, =NOR⁹, =NS(O)ₙN(R¹⁰)(R⁹), =NCN or =C(H)NO₂;
B represents, on each occasion when mentioned above:
I) an aryl group or a heteroaryl group, both of which are optionally substituted by one or more substituents selected from G², methylenedioxy, difluoromethylenedioxy and/or dimethylmethylenedioxy;
II) C₁₋₈ alkyl or a heterocycloalkyl group, both of which are optionally substituted by one or more substituents selected from G² and-/or Z²;
III) a G² group; or
IV) methylenedioxy, difluoromethylenedioxy or dimethylmethylenedioxy;
G² represents, on each occasion when mentioned above, halo, cyano, -N₃, -NO₂, -ONO₂ or -A⁶-R¹¹;
wherein A⁶ represents a single bond or a spacer group selected from -C(O)A⁷-, -S(O)ₙA⁸-, -N(R¹²)A⁹- or -OA¹⁰-, in which:
A⁷ and A⁸ independently represent a single bond, -O, N(R¹²)- or -C(O)-;
A⁹ and A¹⁰ independently represent a single bond, -C(O)-, -C(O)N(R¹²)-, -C(O)O-, S(O)ₙ- or -S(O)ₙN(R¹²)-;
Z² represents, on each occasion when used above, =O, =S; =NOR¹¹, =NS(O)ₙN(R¹²)(R¹¹), =NCN or =C(H)NO₂;
R⁹, R¹⁰, R¹¹ and R¹² are independently selected from:
j) hydrogen;
ii) an aryl group or a heteroaryl group, both of which are optionally substituted by one or more substituents selected from G³, methylenedioxy, difluoromethylenedioxy and/or dimethylmethylenedioxy;
iii) C₁₋₈ alkyl or a heterocycloalkyl group, both of which are optionally substituted by G³ and/or Z³; or
any pair of R⁹ and R¹⁰, or R¹¹ and R¹², may, for example when present on the same or on adjacent atoms, be linked together to form with those, or other relevant, atoms a a further 3- to 8-membered ring, optionally containing 1 to 3 heteroatoms and/or 1 to 3 double bonds, which ring is optionally substituted by one or more substituents selected from G³ and/or Z³;
G³ represents, on each occasion which mentioned above, halo, cyano, -N₃, NO2, -ONO₂ or-A¹¹-R¹³;
wherein A¹¹ represents a single bond or a spacer group selected from -C(O)A¹²-, -S(O)ₙA¹³-, -N(R¹⁴)A¹⁴- or -OA¹⁵-, in which:
A¹² and A¹³ independently represent a single bond, -O-, -N(R¹⁴)- or -C(O)-;
A¹⁴ and A¹⁵ independently represent a single bond, -C(O)-, -C(O)N(R¹⁴)-, -C(O)O-, -S(O)ₙ- or -S(O)ₙN(R¹⁴)-;
Z³ represents, on each occasion when mentioned above, =O, =S, =NOR¹³, =NS(O)ₙN(R¹⁴)(R¹³), =NCN or =C(H)NO₃;
n represents, on each occasion when mentioned above, 1 or 2 ;
R¹³ and R¹⁴ are independently selected from:
i) hydrogen;
ii) C₁₋₆ alkyl or a heterocycloalkyl group, both of which groups are optionally substituted by one or more substituents selected from halo, C₁₋₄ alkyl, -N(R¹⁵)(R¹⁶), -O(R¹⁵) and =O; and
iii) an aryl or heteroaryl group, both of which are optionally substituted by one or more substituents selected from halo, C₁₋₄ alkyl, -N(R¹⁵)(R¹⁶) and -O(R¹³); or
any pair R¹³ and R¹⁴ may, for example when present on the same or on adjacent atoms, be linked together to form with those, or other relevant, atoms a further 3- to 8-membered ring, optionally containing 1 to 3 heteroatoms and/or 1 to 3 double bonds, which ring is optionally substituted by one or more substituents selected from halo, C₁₋₄ alkyl, -N(R¹⁵)(R¹⁶), -O(R¹⁵) and =O;
R¹⁵ and R¹⁶ are independently selected from hydrogen and C₁₋₄ alkyl, which latter group is optionally substituted by one or more halo groups;
or a pharmaceutically-acceptable salt thereof.

2. A compound as claimed in Claim 1, wherein:
A² and A³ independently represent a single bond, -O- or -N(R¹⁰)-;
Z¹ represents, on each occasion when mentioned above, =O, =NOR⁹, =NS(O)ₙN(R¹⁰)(R⁹), =NCN or =C(H)-NO₂;
A⁷ and A⁸ independently represent a single bond, -O- or -N(R¹²)-;
Z² represents, on each occasion when mentioned above, =O, =NOR¹¹, =NS(O)ₙN(R¹²)(R¹¹) =NCN or =C(H)NO₂;
A¹² and A¹³ independently represent a single bond, -O- or -N(R¹⁴)-; and/or Z³ represents, on each occasion when mentioned above, =O, =NOR¹³, =NS(O)ₙN(R¹⁴)(R¹³), =NCN or =C(H)NO₂.

3. A compound as claimed in Claim 1 or Claim 2, wherein n represents 2.

4. A compound as claimed in any one of the preceding claims, wherein 5 X represents a single bond or -C(O)-.

5. A compound as claimed in any one of the preceding claims, wherein A represents G¹.

6. A compound as claimed in any one of the preceding claims, wherein G¹ represents halo or -A¹-R⁹.

7. A compound as claimed in any one of the preceding claim, wherein A¹ represents a single bond, -OA³- or -N(R¹⁰)A¹-.

8. A compound as claimed in any one of the preceding claims, wherein A⁴ and A³ independently represent a single bond.

9. A compound as claimed in any one of the preceding claims, wherein B represents G².

10. A compound as claimed in any one of the preceding claims, wherein G² represents halo or -OR¹¹.

11. A compound as claimed in any one of the preceding claims, wherein R⁹ represents C₁₋₆ alkyl), which group is optionally substituted by one or more halo groups, or a phenyl, or pyridyl group.

12. A compound as claimed in any one of the preceding claims, wherein R¹⁰ represents C₁₋₂alkyl.

13. A compound as claimed in any one of the preceding claims, wherein R¹¹ represents C₁₋₂ alkyl optionally substituted by one or more halo groups.

14. A compound as claimed in any one of the preceding claims, wherein R¹ represents an optionally substituted phenyl or pyridyl group.

15. A compound as claimed in any one of the preceding claims, wherein R³ and R⁴ independently represent G¹, hydrogen or an optionally substituted phenyl or pyridyl group.

16. A compound as claimed in Claim 15, wherein R³ and R⁴ independently represent hydrogen or an optionally substituted phenyl or pyridyl group.

17. A compound as claimed in any one of the preceding claims, wherein at least one of R³ and R⁴ represents optionally substituted phenyl or pyridyl, and up to one other represents G¹ or hydrogen;

18. A compound as claimed in any one of Claims 15 to 17, wherein, when R³ or R⁴ represents an optionally substituted phenyl or pyridyl croup, then the other substituents on the essential benzene ring of the indole of formula 1, as defined in Claim 1, (i.e. R², R⁵ and R³ or R⁴ (as appropriate)) represent hydrogen or G¹.

19. A compound as claimed in any one of the preceding claims, wherein R⁶ represents H or an optionally substituted C₁₋₄ alkyl group.

20. A compound as claimed in any one of the preceding claims, wherein R⁷ represents an optionally substituted C₁₋₄ alkyl group or an optionally substituted phenyl or pyridyl group.

21. A compound as claimed in any one of Claims 1 to 18, wherein R⁶ and R⁷ are linked to form, together with the nitrogen atom and X group to which they are respectively attached, a 5- to 6-membered ring, optionally containing 1 to 2 heteroatoms.

22. A compound as claimed in any one of Claims 14 to 20, wherein the optional substituents are selected from cyano, halo) C₁₋₆ alkyl, which alkyl group may be linear or branched cyclic, part-cyclic, unsaturated and/or optionally substituted with one or more halo group, and -OR¹⁷, wherein R¹⁷ represents, H or C₁₋₆ alkyl (which alkyl group is optionally substituted by one or more halo groups).

23. A compound as claimed in Claim 22, wherein the optional substituents are selected from halo, C₁₋₆ alkyl, which alkyl] group may be linear or branched, cyclic, part-cyclic, unsaturated and/or optionally substituted with one or more halo group, and -OR¹⁷, wherein R¹⁷ represents, H or C₁₋₆ alkyl (which alkyl group is optionally substituted by one or more halo groups).

24. A compound as claimed in any one of the preceding claims, wherein R⁸ represents hydrogen.

25. A compound as defined in any one of Claims 1 to 24, or a pharmaceutically-acceptable salt thereof, for use as a pharmaceutical.

26. A pharmaceutical formulation including a compound as defined in any one of Claims 1 to 24 or a pharmaceutically-acceptable salt thereof, in admixture with a pharmaceutically acceptable adjuvant, diluent or carrier.

27. The use of a compound as defined in any one of Claims 1 to 24, or a pharmaceutically-acceptable salt thereof, for the manufacture of a medicament for the treatment of a disease in which inhibition of the activity of microsomal prostaglandin E synthase-1, leukotriene C₄ synthase and/or 5-lipoxygenase-activaing protein is desired and/or required.

28. A use as claimed in Claim 27, wherein inhibition of the activity of microsomal prostaglandin E synthase-1 is desired and/or required.

29. A use as claimed in Claim 27 or Claim 28, wherein the disease is inflammation.

30. A use as claimed in Claim 29 wherein the disease is inflammatory bowel disease, irritable bowel syndrome, migraine, headache, low back pain, fibromyalgia, a myofascial disorder, a viral infection, a bacterial infection, a fungal infection, dysmenorrhea, a burn, a surgical or dental procedure, a malignancy, atherosclerosis, gout, arthritis, osteoarthritis, juvenile arthritis, rheumatoid arthritis, fever, ankylosing spondylitis, systemic lupus erythematosus, vasculitis, pancreatitis, nephritis, bursitis, conjunctivitis, iritis, scleritis, uveitis, wound healing, dermatitis, eczema, psoriasis, stroke, diabetes mellitus, a neurodegenerative disorder, an autoimmune disease, osteoporosis, asthma, chronic obstructive pulmonary disease, pulmonary fibrosis, an allergic disorder, rhinitis, an ulcer, corouary heart disease, sarcoidosis, inflammatory pain, hyperprostaglandin E syndrome, classic Bartter syndrome, Hodgkin's disease, persistent ductus, any other disease with an inflammatory component Pager's disease or a periodontal disease.

31. A combination product comprising:
(A) a compound as defined in any one of Claims 1 to 24, or a pharmaceutically-acceptable salt thereof; and
(B) another therapeutic agent that is useful in the treatment of inflammation
wherein each of components (A) and (B) is formulated in admixture with a pharmaceutically-acceptable adjuvant, diluent or carrier.

32. A combination product as claimed in Claim 31 which comprises a pharmaceutical formulation including a compound as defined in any one of Claims 1 to 24, or a pharmaceutically-acceptable salt thereof, another therapeutic agent that is useful in the treatment of inflammation, and a pharmaceutically-acceptable adjuvant, diluent or carrier.

33. A combination product as claimed in Claim 31 which comprises a kit of parts comprising components:
(a) a pharmaceutical formulation including a compounds as defined in any one of Claims 1 to 24, or a pharmaceutically-acceptable salt thereof, in admixture with a pharmaceutically-acceptable adjuvant, diluent or carrier; and
(b) a pharmaceutical formulation including another therapeutic agent that is useful in the treatement of inflammation in admixture with a pharmaceutically-acceptable adjuvant, diluent or carrier,
which components (a) and (b) are each provided in a form that is suitable for administration in conjunction with the other.

34. A process for the preparation of a compound as defined in Claim 1, which comprises:
(i) reaction of a compound of formula II, 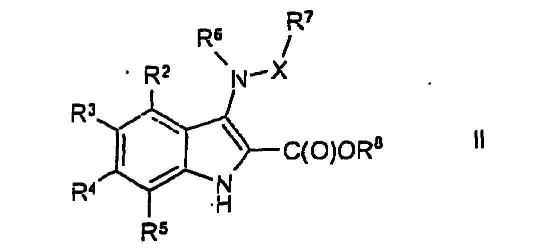 wherein X, R², R³, R⁴, R⁵, R⁶, R⁷ and R⁸ are as defined in Claim 1, with a compound of formula III,
R¹L¹ III
wherein L¹ represents a suitable leaving group and R¹ is as defined in Claim 1;
(ii) reaction of a compound of formula IV, 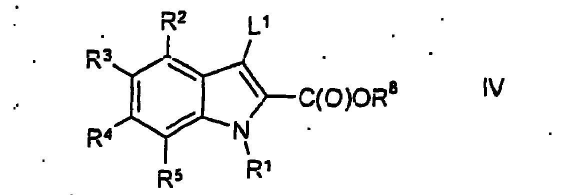 wherein L¹ is as defined above and R¹, R², R³, R⁴, R⁵ and R¹ are as defined in Claim 1, with a compound of fonnula V,
HN(R⁶)XR⁷ V
wherein X, R⁶ and R⁷ are as defined in Claim 1 ;
(iii) reaction of a compound of formula V1, 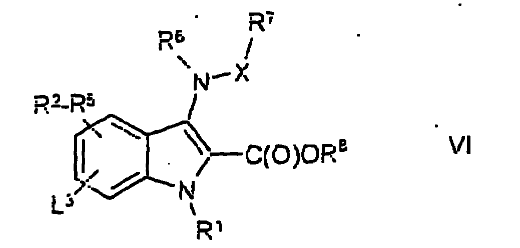 wherein L³ represents L¹ or L², in which L² represents a suitable leaving group and is attached to one or more of the carbon atoms of the benzenoid ring of the indole, and wherein the remaining positions of the benzenoid ring are substituted with I to 3 (depending on the number of L³ substituents) R²-R³ substituents, R²-R⁵ represents any one of the substituents, i.e. R², R³, R⁴ and R⁵, that are already present in that ring (as appropriate), L¹ is as defined above and X, R², R², R³, R⁴, R⁵, R⁶, R⁷ and R⁸ are as defined in Claim 1, with a compound of formula VII,
R¹⁸ L⁴ VII
wherein R¹⁸ represents R², R³, R⁴ or R⁵ (as appropriate) and L⁴ represents L¹ (when L³ is L²) or L² (when L³ is L¹) as defined above,
(iv) reaction of a compound of formula VIII. 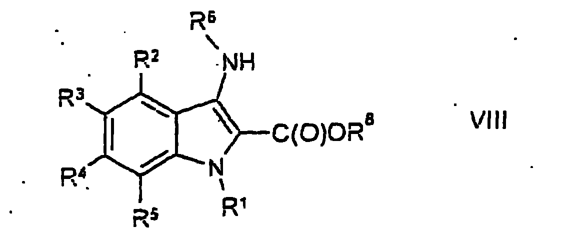 wherein R¹, R², R³, R⁴, R⁵, R⁶ and R⁸ are as defined in Claim 1, with a compound of formula IX,
R⁷XL¹ IX
wherein L¹ is as defined above and X and R⁷ are as defined in Claim 1; or
(v) for compounds of formula I wherein X represents a single bond and R⁷ is a C₁₋₈ alkyl group, reduction of a compound of formula 1, wherein X represents -C(O)- and R⁷ represents H or a C₁₋₇ alkyl group.

## Patentansprüche

1. Eine Verbindung gemäß Formel I, 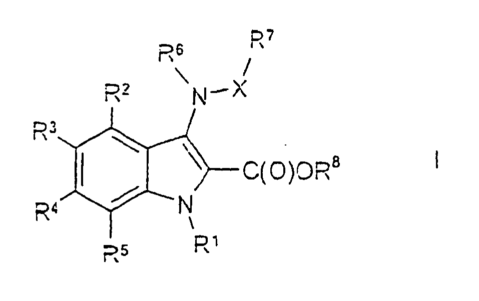 in welcher
X eine Einfachbindung, -C(O)- oder -S(O)₂- bedeutet;
R¹ eine Aryl-Gruppe oder eine Heteroaryl-Gruppe bedeutet, wobei beide Gruppen optional durch einen oder mehrere Substituenten substituiert sind, die aus A ausgewählt sind,
eine der Gruppen R², R³, R⁴ oder R⁵ eine Aryl-Gruppe oder eine Heteroaryl-Gruppe bedeutet (, von denen beide optional durch einen oder mehrere aus A ausgewählten Substituenten substituiert sind), und:
a) die anderen Gruppen unabhängig voneinander ausgewählt sind aus Wasserstoff, G¹, einer Aryl-Gruppe, einer Heteroaryl-Gruppe (wobei die beiden letztgenannten Gruppen optional durch einen oder mehrere aus A ausgewählte Substituenten substituiert sind), C₁₋₈-Alkyl- oder einer Heterocycloalkyl-Gruppe (wobei die beiden letztgenannten Gruppen optional durch einen oder mehrere Substituenten substituiert sind, die aus G¹ und/oder Z¹ ausgewählt sind); und/oder
b) irgend zwei andere Gruppen, die einander benachbart sind, optional verbunden sind, um zusammen mit zwei Atomen des essentiellen Benzen-Rings in der Verbindung gemäß Formel **I** einen 3- bis 8-gliedrigen Ring zu bilden, der optional 1 bis 3 Heteroatome und/oder 1 bis 3 Doppelbindungen enthält, wobei der Ring selbst optional durch einen oder mehrere Substituenten substituiert ist, die aus Halo, -R⁸, - OR⁸ und =O ausgewählt sind;
R⁶, R⁷ und R⁸ unabhängig voneinander immer dort, wo sie oben verwendet werden:
I) Wasserstoff;
II) eine Aryl-Gruppe oder eine Heteroaryl-Gruppe, von denen beide optional durch einen oder mehrere Substituenten substituiert sind, die aus B ausgewählt sind; oder;
III) C₁₋₈-Alkyl oder eine Heterocycloalkyl-Gruppe bedeuten, von denen beide optional durch einen oder mehrere Substituenten substituiert sind, die aus G¹ und/oder Z¹ ausgewählt sind, oder
R⁶ und R⁷ verknüpft sein können, um zusammen mit dem N-Atom und der X-Gruppe, an welche R⁶ und R⁷ jeweils angehängt sind, einen 3- bis 8-gliedrigen Ring bilden, der optional 1 bis 3 Heteroatome und/oder 1 bis 3 Doppelbindungen enthält,
wobei dieser Ring optional durch einen oder mehrere Substituenten substituiert ist, die aus G¹ und/oder Z¹ ausgewählt sind, wobei
A immer dort, wo es oben erwähnt wird:
I) eine Aryl-Gruppe oder eine Heteroaryl-Gruppe, von denen beide optional durch einen oder mehrere aus B ausgewählte Substituenten substituiert sind;
II) C₁₋₈-Alkyl oder eine Heterocycloalkyl-Gruppe, von denen beide optional durch einen oder mehrere Substituenten substituiert sind, die aus G¹ und/oder Z¹ ausgewählt sind;
III) oder eine G¹-Gruppe bedeutet; oder
IV) zwei A-Substituenten miteinander verknüpft sein können, um gemeinsam mit wenigstens zwei (z.B. benachbarten) Atomen der Aryl- oder Heteroaryl-Gruppe, an welche die beiden A-Substituenten angehängt sind, einen weiteren 3- bis 5-gliedrigen Ring zu bilden, wobei dieser Ring optional 1 bis 3 Heteroatome und/oder 1 bis 2 Doppelbindungen enthält, und optional durch Halo oder C₁₋₈-Alkyl substituiert ist,
wobei die letztgenannte Gruppe optional durch Halo substituiert ist;
G¹ immer dort, wo es oben erwähnt wird, Halo, Cyano, -N₃, -NO₂, -ONO₂ oder -A¹-R⁹ bedeutet,
wobei A¹ eine Einzelbindung oder Abstandsgruppe bedeutet, die aus -C(O)A²-, - S(O)ₙA³, -N(R¹⁰)A⁴- oder -OA⁵- ausgewählt ist, wobei:
A² und A³ unabhängig voneinander eine Einzelbindung, -O-, -N(R¹⁰)- oder -C(O)-bedeuten und
A⁴ und A⁵ unabhängig voneinander eine Einzelbindung, -C(O)-, -C(O)N(R¹⁰)-, -C(O)O-, -S(O)ₙ- oder -S(O)ₙN(R¹⁰)- bedeuten;
Z¹ überall dort, wo es oben erwähnt wird, =O, =S, =NOR⁹, =NS(O)ₙN(R¹⁰)(R⁹), =NCN oder =C(H)NO₂ bedeutet;
B überall dort, wo es oben erwähnt wird:
I) eine Aryl-Gruppe oder eine Heteroaryl-Gruppe, von denen beide optional durch einen oder mehrere Substituenten substituiert sein können, die aus G², Methylendioxy, Difluoromethylendioxy und/oder Dimethylmethylendioxy;
II) eine C₁₋₈-Alkyl- oder eine Heterocycloalkyl-Gruppe, von denen beide optional durch einen oder mehrere Substituenten substituiert sind, die aus G² und/oder Z² ausgewählt sind;
III) eine G²-Gruppe; oder
IV) Methylendioxy, Difluoromethylendioxy oder Dimethylmethylendioxy bedeutet;
G² überall dort, wo es oben erwähnt wird, Halo, Cyano, -N₃,-NO₂, -ONO₂ oder -A⁶-R¹¹ bedeutet,
wobei A⁶ eine Einzelbindung oder eine Abstandsgruppe bedeutet, die aus -C(O)A⁷-, -S(O)ₙA⁸-, -N(R¹²)A⁹- oder -OA¹⁰- ausgewählt ist, wobei:
A⁷ und A⁸ unabhängig voneinander eine Einzelbindung, -O-, -N(R¹²)- oder -C(O)-bedeuten; und
A⁹ und A¹⁰ unabhängig voneinander eine Einzelbindung, -C(O)-, -C(O)N(R¹²)-, -C(O)O-, -S(O)ₙ- oder -S(O)ₙN(R¹²)- bedeuten;
Z² überall dort, wo es oben verwendet wird, =O, =S, =NOR¹¹, =NS(O)ₙN(R¹²)(R¹¹), =NCN oder =C(H)NO₂ bedeutet;
R⁹, R¹⁰, R¹¹ und R¹² voneinander unabhängig ausgewählt sind aus:
i) Wasserstoff;
ii) einer Aryl-Gruppe oder einer Heteroaryl-Gruppe, von denen beide optional durch einen oder mehrere Substituenten substituiert sein können, die aus G³, Methylendioxy, Difluoromethylendioxy und/oder Dimethylmethylendioxy ausgewählt sind;
iii) einer C₁₋₈-Alkyl- oder eine Heterocycloalkyl-Gruppe, von denen beide optional durch G³ und/oder Z³ substituiert sind; oder wobei
irgendein Paar von R⁹ und R¹⁰, oder R¹¹ und R¹², beispielsweise dann, wenn sie an dem gleichen oder an benachbarten Atomen vorhanden sind, miteinander verknüpft sein können, um mit diesen oder anderen relevanten Atomen einen weiteren 3- bis 8-gliedrigen Ring zu bilden, der optional 1 bis 3 Heteroatome und/oder 1 bis 3 Doppelbindungen enthält, wobei dieser Ring optional durch einen oder mehrere Substituenten substituiert ist, die aus G³ und/oder Z³ ausgewählt sind;
G³ überall dort, wo es oben erwähnt wird Halo, Cyano, -N₃, -NO₂, -ONO₂ oder -A¹¹-R¹³ bedeutet;
wobei A¹¹ eine Einzelbindung oder eine Abstands-Gruppe bedeutet, die aus -C(O)A¹²-, -S(O)ₙA¹³-, -N(R¹⁴)A¹⁴- oder -OA¹⁵- ausgewählt ist, wobei:
A¹² und A¹³ unabhängig voneinander eine Einzelbindung, -O-, -N(R¹⁴)- oder -C(O)-bedeuten; und
A¹⁴ und A¹⁵ unabhängig voneinander eine Einzelbindung, -C(O)-, -C(O)N(R¹⁴)-, -C(O)O-, -S(O)ₙ- oder -S(O)ₙN(R¹⁴)- bedeuten;
Z³ überall dort, wo es oben erwähnt wird, =O, =S, =NOR¹³, =NS(O)ₙN(R¹⁴)(R¹³), =NCN oder =C(H)NO₂ bedeutet;
n überall dort, wo es oben erwähnt wird, entweder 1 oder 2 bedeutet;
R¹³ und R¹⁴ unabhängig voneinander ausgewählt sind aus:
i) Wasserstoff;
ii) einer C₁₋₆-Alkyl- oder einer Heterocycloalkyl-Gruppe, von denen beide Gruppen optional durch einen oder mehrere Substituenten substituiert sind, die aus Halo, C₁₋₄-Alkyl, -N(R¹⁵)(R¹⁶), -O(R¹⁵) und =O ausgewählt sind; und
iii) einer Aryl- oder einer Heteroaryl-Gruppe, von denen beide optional durch einen oder mehrere Substituenten substituiert sind, die aus Halo, C₁₋₄-Alkyl, N(R¹⁵)(R¹⁶) und -O (R¹⁵) ausgewählt sind; wobei
jedes R¹³ und R¹⁴ dann, wenn sie beispielsweise an den gleichen oder an benachbarten Atomen paarweise vorhanden sind, miteinander verbunden sein können, um mit diesen oder anderen relevanten Atomen einen weiteren 3- bis 8-gliedrigen Ring zu bilden, der optional 1 bis 3 Heteroatome und/oder 1 bis 3 Doppelbindungen enthält, wobei dieser Ring optional durch einen oder mehrere Substituenten substituiert ist, die aus Halo, C₁₋₄-Alkyl, -N(R¹⁵)(R¹⁶), -O(R¹⁵) und =O ausgewählt sind; und wobei
R¹⁵ und R¹⁶ unabhängig voneinander ausgewählt sind aus Wasserstoff und C₁₋₄-A1-kyl, wobei die letztgenannte Gruppe optional durch eine oder mehrere Halo-Gruppen substituiert ist,
oder ein pharmazeutisch akzeptables Salz hiervon.

2. Verbindung nach Anspruch 1, bei der
A² und A³ unabhängig voneinander eine Einzelbindung, -O- oder -N(R¹⁰)- bedeuten;
Z¹ überall dort, wo es oben erwähnt wird, =O, =NOR⁹, =NS(O)ₙN(R¹⁰)(R⁹), =NCN oder =C(H)NO₂ bedeutet;
A⁷ und A⁸ unabhängig voneinander eine Einzelbindung, -O- oder -N(R¹²)- bedeuten;
Z² überall dort, wo es oben erwähnt wird, =O, =NOR¹¹, =NS(O)ₙN(R¹²)(R¹¹), =NCN oder =C(H)NO₂ bedeutet;
A¹² und A¹³ unabhängig voneinander eine Einzelbindung, -O- oder -N(R¹⁴ )- bedeuten; und/oder
Z³ überall dort, wo es oben erwähnt wird, =O, =NOR¹³, =NS(O)ₙN(R¹⁴)(R¹³), =NCN oder =C(H)NO₂ bedeutet.

3. Verbindung nach Anspruch 1 oder 2, bei der n gleich 2 ist.

4. Verbindung nach einem der vorhergehenden Ansprüche, bei der X eine Einzelbindung oder -C(O)- bedeutet.

5. Verbindung nach einem der vorhergehenden Ansprüche, bei der A für G¹ steht.

6. Verbindung nach einem der vorhergehenden Ansprüche, bei der G¹ für Halo oder -A¹-R⁹ steht.

7. Verbindung nach einem der vorhergehenden Ansprüche, bei der A¹ eine Einzelbindung, -OA⁵- oder -N(R¹⁰)A⁴- bedeutet.

8. Verbindung nach einem der vorhergehenden Ansprüche, bei der A⁴ und A⁵ unabhängig voneinander eine Einzelbindung bedeuten.

9. Verbindung nach einem der vorhergehenden Ansprüche, bei der B für G² steht.

10. Verbindung nach einem der vorhergehenden Ansprüche, bei der G² für Halo oder -OR¹¹ steht.

11. Verbindung nach einem der vorhergehenden Ansprüche, bei der R⁹ für C₁₋₆-Alkyl steht, wobei diese Gruppe optional durch eine oder mehrere Halo-Gruppen oder eine Phenyl- oder Pyridyl-Gruppe substituiert ist.

12. Verbindung nach einem der vorhergehenden Ansprüche, bei der R¹⁰ für C₁₋₂-Alkyl steht.

13. Verbindung nach einem der vorhergehenden Ansprüche, bei der R¹¹ für C₁₋₁₂-Alkyl steht, das optional durch eine oder mehrere Halo-Gruppen substituiert ist.

14. Verbindung nach einem der vorhergehenden Ansprüche, bei der R¹ eine optional substituierte Phenyl- oder Pyridyl-Gruppe bedeutet.

15. Verbindung nach einem der vorhergehenden Ansprüche, bei der R³ und R⁴ unabhängig voneinander G¹, Wasserstoff oder eine optional substituierte Phenyl- oder Pyridyl-Gruppe bedeuten.

16. Verbindung nach Anspruch 15, bei der R³ und R⁴ voneinander unabhängig Wasserstoff oder eine optional substituierte Phenyl- oder Pyridyl-Gruppe bedeuten.

17. Verbindung nach einem der vorhergehenden Ansprüche, bei der wenigstens eines von R³ und R⁴ optional substituiertes Phenyl oder Pyridyl bedeutet und bis zu ein anderes G¹ oder Wasserstoff bedeutet.

18. Verbindung nach einem der Ansprüche 15 bis 17, bei der dann, wenn R³ oder R⁴ eine optional substituierte Phenyl- oder Pyridyl-Gruppe bedeutet, die anderen Substituenten an dem essentiellen Benzen-Ring des Indols aus Formel I, wie in Anspruch 1 definiert (d.h. R², R⁵ und R³ oder R⁴ (gegebenenfalls)) Wasserstoff oder G¹ bedeuten.

19. Verbindung nach einem der vorhergehenden Ansprüche, bei der R⁶ für H oder eine optional substituierte C₁₋₄-Alkyl-Gruppe steht.

20. Verbindung nach einem der vorhergehenden Ansprüche, bei der R⁷ eine optional substituierte C₁₋₄-Alkyl-Gruppe oder eine optional substituierte Phenyl- oder Pyridyl-Gruppe bedeutet.

21. Verbindung nach einem der Ansprüche 1 bis 18, bei der R⁶ und R⁷ verknüpft sind, um zusammen mit dem Stickstoff-Atom und der X-Gruppe an die sie jeweils gebunden sind, einen 5- bis 6-gliedrigen Ring zu bilden, der optional 1 bis 2 Heteroatome enthält.

22. Verbindung nach einem der Ansprüche 14 bis 20, bei der die optionalen Substituenten aus folgenden Gruppen ausgewählt sind: Cyano, Halo, C₁₋₆-Alkyl, wobei diese Alkyl-Gruppe linear oder verzweigt, zyklisch, teil-zyklisch, ungesättigt und/oder optional mit einer oder mehreren Halo-Gruppen substituiert sein kann, und -OR¹⁷,
wobei R¹⁷ für H oder C₁₋₆-Alkyl steht (wobei diese Alkyl-Gruppe optional durch eine oder mehrere Halo-Gruppen substituiert ist).

23. Verbindung nach Anspruch 22, bei der die optionalen Substituenten aus folgenden Gruppen ausgewählt sind: Halo, C₁₋₆-Alkyl, wobei diese Alkyl-Gruppe linear oder verzweigt, zyklisch, teil-zyklisch, ungesättigt und/oder optional mit einer oder mehreren Halo-Gruppen substituiert sein kann, und -OR¹⁷, wobei R¹⁷ für H oder C₁₋₆-Alkyl steht (, wobei diese Alkyl-Gruppe optional durch eine oder mehrere Halo-Gruppen substituiert ist).

24. Verbindung nach einem der vorhergehenden Ansprüche, bei der R⁸ für Wasserstoff steht.

25. Verbindung nach einem der Ansprüche 1 bis 24, oder pharmazeutisch akzeptables Salz hiervon zur Verwendung als Pharmazeutikum.

26. Pharmazeutische Zubereitung, die eine Verbindung umfasst, wie sie in einem der Ansprüche 1 bis 24 definiert ist, oder ein pharmazeutisch akzeptables Salz hiervon in Mischung mit einem pharmazeutisch akzeptablen Zusatzstoff, Verdünner oder Träger.

27. Verwendung einer Verbindung nach einem der Ansprüche 1 bis 24, oder eines pharmazeutisch akzeptablen Salzes hiervon für die Herstellung eines Medikamentes zur Behandlung einer Erkrankung, bei der eine Hemmung der Aktivität von mikrosomaler Prostaglandin-E-Synthase-1, von Leukotrien-C₄-Synthase und/oder einem 5-Lipoxygenase aktivierendem Protein erwünscht und/oder erforderlich ist.

28. Verwendung nach Anspruch 27, bei der die Hemmung der Aktivität von mikrosomaler Prostaglandin-E-Synthase-1 erwünscht und/oder erforderlich ist.

29. Verwendung nach Anspruch 27 oder 28, wobei die Erkrankung eine Entzündung ist.

30. Verwendung nach Anspruch 29, wobei die Erkrankung eine entzündliche Darm-Erkrankung, das Reizdarm-Syndrom, Migräne, Kopfschmerzen, Schmerzen im unteren Rücken, Fibromyalgie, eine myofasciale Erkrankung, eine Virusinfektion, eine bakterielle Infektion, eine Pilzinfektion, Dysmenorrhö, eine Verbrennung, eine chirurgische Behandlung oder Zahnbehandlung, eine bösartige Erkrankung, Atherosklerose, Gicht, Arthritis, Osteoarthritis, jugendliche Arthritis, rheumatoide Arthritis, Fieber, Spondylitis ankylosans, systemischer Lupus erythematosus, eine Gefäßentzündung, eine Entzündung der Bauchspeicheldrüse, eine Nierenentzündung, eine Schleimbeutelentzündung, eine Bindehautentzündung, eine Regenbogenhautentzündung, eine Scleritis, eine Augenhaut-Entzündung, eine Wundheilungs-Dermatitis, ein Ekzem, eine Psoriasis, ein Schlaganfall, Diabetes mellitus, eine neurodegenerative Erkrankung, eine Autoimmun-Erkrankung, Osteoporose, Asthma, eine chronische obstruktive Pulmonar-Erkrankung, Pulmonar-Fibrose, eine allergische Erkrankung, eine Nasenschleimhaut-Entzündung, ein Geschwür, eine Herzkranz-Erkrankung, Sarkoidose, Entzündungsschmerzen, das Hyperprostaglandin-E-Syndrom, das klassische Bartter-Syndrome, die Hodgkins Erkrankung, ein persistierender Ductus, irgendeine andere Erkrankung mit einer entzündlichen Komponente, Paget's Erkrankung oder eine periodontale Erkrankung ist.

31. Kombinationsprodukt, das Folgendes umfasst:
(A) Eine Verbindung nach einem Ansprüche 1 bis 24, oder ein pharmazeutisch akzeptables Salz hiervon; und
(B) einen weiteren therapeutischen Wirkstoff, der bei der Behandlung von Entzündungen nützlich ist,
wobei jede der Komponenten (A) und (B) in Mischung mit einem pharmazeutisch akzeptablen Zusatzstoff, Verdünner oder Träger zubereitet ist.

32. Kombinationsprodukt nach Anspruch 31, das eine pharmazeutische Zubereitung umfasst, die eine Verbindung nach einem der Ansprüche 1 bis 24, oder ein pharmazeutisch akzeptables Salz hiervon, einen weiteren therapeutischen Wirkstoff, der bei der Behandlung von Entzündungen nützlich ist, und einen pharmazeutisch akzeptablen Zusatzstoff, Verdünner oder Träger umfasst.

33. Kombinationsprodukt nach Anspruch 31, das einen Teilesatz umfasst, der folgende Komponenten aufweist:
(a) eine pharmazeutische Zubereitung, die eine Verbindung nach einem der Ansprüche 1 bis 24, oder ein pharmazeutisch akzeptables Salz hiervon in Mischung mit einem pharmazeutisch akzeptablen Zusatzstoff, Verdünner oder Träger umfasst, und
(b) eine pharmazeutische Zubereitung, die einen anderen therapeutischen Wirkstoff umfasst, der bei der Behandlung von Entzündungen nützlich ist, in Mischung mit einem pharmazeutisch akzeptablen Zusatzstoff, Verdünner oder Träger,
wobei die Komponenten (a) und (b) jeweils in einer Form vorgesehen sind, die für eine Verabreichung in Verbindung mit der anderen geeignet ist.

34. Verfahren für die Zubereitung einer Verbindung nach Anspruch 1, das Folgendes umfasst:
(i) Reaktion einer Verbindung gemäß Formel II, 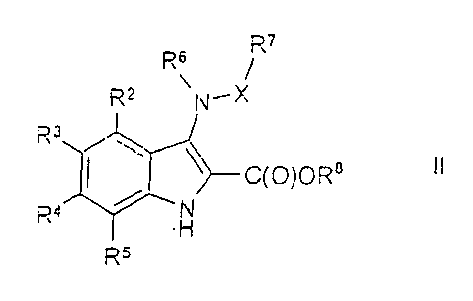 wobei X, R², R³, R⁴, R⁵, R⁶, R⁷und R⁸ der Definition in Anspruch 1 entsprechen, mit einer Verbindung gemäß Formel III
R¹L¹ (III)
wobei L¹ eine geeignete verlassende Gruppe darstellt und R¹ der Definition in Anspruch 1 entspricht;
(ii) Reaktion einer Verbindung gemäß der Formel IV,
wobei L¹ der obigen [ 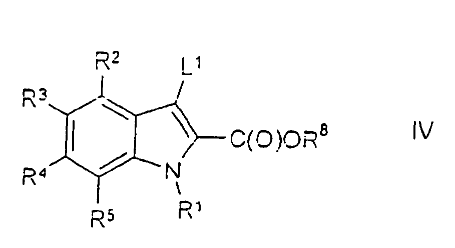 R⁸ der Definition in Anspruch 1 ents
HN(R⁶)XR⁷ V
wobei X, R⁶ und R⁷ der Definition in Anspruch 1 entsprechen;
(iii) Reaktion einer Verbindung nach Formel VI, 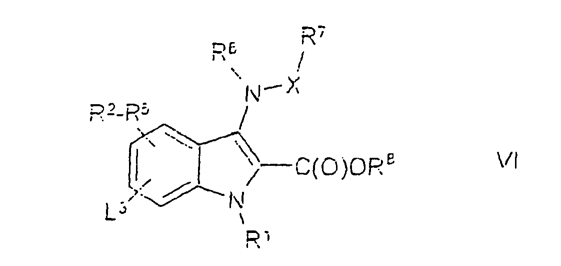 wobei L³ für L¹ oder L² steht, wobei L² eine geeignete verlassende Gruppe darstellt und an eines oder mehrere der Kohlenstoffatome des Benzenoid-Rings des Indols angehängt ist, und wobei die übrigen Positionen des Benzenoid-Rings mit 1 bis 3 (abhängig von der Anzahl der L³ Substituenten) R²-R⁵-Substituenten substituiert sind, wobei R²-R⁵ irgend einen der Substituenten bedeutet, d.h. R², R³, R⁴ und R⁵, die bereits in diesem Ring (gegebenenfalls) vorhanden sind, wobei L¹ der obigen Definition entspricht und X, R¹, R², R³, R⁴, R⁵, R⁶, R⁷ und R⁸ der Definition in Anspruch 1 entsprechen, mit einer Verbindung gemäß der Formel VII,
R¹⁸L⁴ VII
wobei R¹⁸ für R², R³, R⁴ oder R⁵ (gegebenenfalls) steht und L⁴ für L¹ steht (wenn L³ gleich L² ist) oder für L² (wenn L³ gleich L¹ ist) wie oben definiert;
(iv) Reaktion einer Verbindung gemäß Formel VIII, 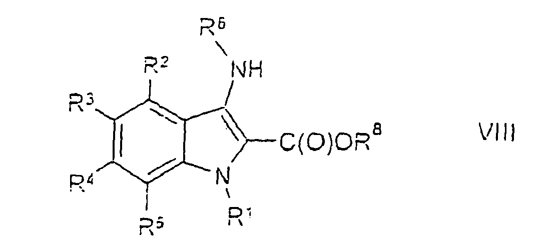 wobei R¹, R², R³, R⁴, R⁵, R⁶ und R° der Definition in Anspruch 1 entsprechen, mit einer Verbindung der Formel IX,
R⁷XL¹ (IX)
wobei L¹ der obigen Definition entspricht und X und R⁷ der Definition in Anspruch 1 entsprechen; oder
(v) bei Verbindungen der Formel I, bei denen X für eine Einzelbindung steht und R⁷ eine C₁₋₈-Alkyl-Gruppe bedeutet, Reduktion einer Verbindung der Formel I, wobei X für -C(O)- steht und R⁷ für H oder eine C₁₋₇-Alkyl-Gruppe steht.

## Revendications

1. Composé de formule I, 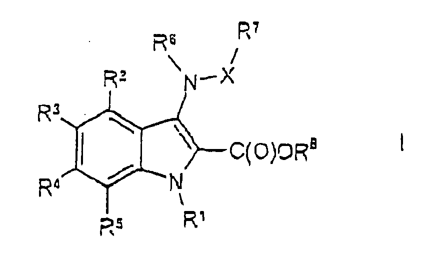 dans laquelle
X représente une liaison simple, -C(O)- ou -S(O)₂- ;
R¹ représente un groupe aryle ou un groupe hétéroaryle, ces deux groupes étant facultativement substitués par un ou plusieurs substituants choisis parmi A ;
l'un des groupes R², R³, R⁴ et R⁵ représente un groupe aryle ou un groupe hétéroaryle (ces deux groupes étant facultativement substitués par un ou plusieurs substituants choisis parmi A) et :
a) les autres groupes sont indépendamment choisis parmi un hydrogène, G¹, un groupe aryle, un groupe hétéroaryle (ces deux derniers groupes étant facultativement substitués par un ou plusieurs substituants choisis parmi A), un alkyle en C₁₋₈ et un groupe hétérocycloalkyle (ces deux derniers groupes étant facultativement substitués par un ou plusieurs substituants choisis parmi G¹ et/ou Z¹) ; et/ou
b) deux quelconques autres groupes qui sont adjacents entre eux sont facultativement liés pour former, conjointement avec deux atomes du cycle benzénique essentiel dans le composé de formule I, un cycle à 3 à 8 chaînons, contenant facultativement 1 à 3 hétéroatomes et/ou 1 à 3 liaisons doubles, lequel cycle est lui-même facultativement substitué par un ou plusieurs substituants choisis parmi un halogéno, -R⁸, -OR⁸ et =O ;
R⁶, R⁷ et R⁸ représentent indépendamment, à chaque fois qu'ils sont utilisés ci-dessus :
I) un hydrogène ;
II) un groupe aryle ou un groupe hétéroaryle, ces deux groupes étant facultativement substitués par un ou plusieurs substituants choisis parmi B ;
III) un alkyle en C₁₋₈ ou un groupe hétérocycloalkyle, ces deux groupes étant facultativement substitués par un ou plusieurs substituants choisis parmi G¹ et/ou Z¹ ; ou
R⁶ et R⁷ peuvent être liés ensemble pour former, conjointement avec l'atome N et le groupe X auxquels R⁶ et R⁷ sont respectivement attachés, un cycle à 3 à 8 chaînons, contenant facultativement 1 à 3 hétéroatomes et/ou 1 à 3 liaisons doubles, lequel cycle est facultativement substitué par un ou plusieurs substituants choisis parmi G¹ et/ou Z¹ ;
A représente, à chaque fois qu'il est mentionné ci-dessus :
I) un groupe aryle ou un groupe hétéroaryle, ces deux groupes étant facultativement substitués par un ou plusieurs substituants choisis parmi B ;
II) un alkyle en C₁₋₈ ou un groupe hétérocycloalkyle, ces deux groupes étant facultativement substitués par un ou plusieurs substituants choisis parmi G¹ et/ou Z¹ ;
III) un groupe G¹ ; ou
IV) deux substituants A peuvent être liés ensemble pour former, conjointement avec au moins deux atomes (par exemple, adjacents) du groupe aryle ou hétéroaryle auquel les deux substituants A sont attachés, un autre cycle à 3 à 5 chaînons, lequel cycle contient facultativement 1 à 3 hétéroatomes et/ou 1 à 2 liaisons doubles, et qui est facultativement substitué par un halogéno ou un alkyle en C₁₋₈, ce dernier groupe étant facultativement substitué par un halogéno ;
G¹ représente, à chaque fois qu'il est mentionné ci-dessus, un halogéno, un cyano, -N₃, -NO₂, -ONO₂ ou -A¹-R⁹ ;
où A représente une liaison simple ou un groupe espaceur choisi parmi -C(O)A²-, -S(O)ₙA³-, -N(R¹⁰)A⁴- ou -OA⁵-, dans lesquels :
A² et A³ représentent indépendamment une liaison simple, -O-, -N(R¹⁰)- ou -C(O) - ;
A⁴ et A⁵ représentent indépendamment une liaison simple, -C(O)-, -C(O)N(R¹⁰)-, -C(O)O-, -S(O)ₙ- ou -S(O)ₙN(R¹⁰)- ;
Z¹ représente, à chaque fois qu'il est mentionné ci-dessus, =O, =S, =NOR⁹, =NS(O)ₙN(R¹⁰)(R⁹), =NCN ou =C(H)NO₂ ;
B représente, à chaque fois qu'il est mentionné ci-dessus :
I) un groupe aryle ou un groupe hétéroaryle, ces deux groupes étant facultativement substitués par un ou plusieurs substituants choisis parmi G², un méthylènedioxy, un difluorométhylènedioxy et/ou un diméthylméthylènedioxy ;
II) un alkyle en C₁₋₈ ou un groupe hétérocycloalkyle, ces deux groupes étant facultativement substitués par un ou plusieurs substituants choisis parmi G² et/ou Z² ;
III) un groupe G² ; ou
IV) un méthylènedioxy, un difluorométhylènedioxy ou un diméthylméthylènedioxy ;
G² représente, à chaque fois qu'il est mentionné ci-dessus, un halogéno, un cyano, -N₃, -NO₂, -ONO₂ ou -A⁶-R¹¹ ;
où A⁶ représente une liaison simple ou un groupe espaceur choisi parmi -C(O)A⁷-, -S(O)ₙA⁸-, -N(R¹²)A⁹- ou -OA¹⁰-, dans lesquels :
A⁷ et A⁸ représentent indépendamment une liaison simple, -O-, -N(R¹²) - ou -C(O)- ;
A⁹ et A¹⁰ représentent indépendamment une liaison simple, -C (O) -, -C(O)N(R¹²)-, -C (O) O-, -S(O)ₙ- ou -S(O)ₙN(R¹²)- ;
Z² représente, à chaque fois qu'il est utilisé ci-dessus, =O, =S, =NOR¹¹, =NS(O)ₙN(R¹²) (R¹¹), =NCN ou =C(H)NO₂ ;
R⁹, R¹⁰, R¹¹ et R¹² sont indépendamment choisis parmi :
i) un hydrogène ;
ii) un groupe aryle ou un groupe hétéroaryle, ces deux groupes étant facultativement substitués par un ou plusieurs substituants choisis parmi G³, un méthylènedioxy, un difluorométhylènedioxy et/ou un diméthylméthylènedioxy ;
iii) un alkyle en C₁₋₈ ou un groupe hétérocycloalkyle, ces deux groupes étant facultativement substitués par G³ et/ou Z³ ; ou
toute paire parmi R⁹ et R¹⁰, ou R¹¹ et R¹², peut, par exemple, lorsqu'elle est présente sur des atomes identiques ou adjacents, être liée ensemble pour former avec ces atomes, ou d'autres appropriés, un autre cycle à 3 à 8 chaînons, contenant facultativement 1 à 3 hétéroatomes et/ou 1 à 3 liaisons doubles, lequel cycle est facultativement substitué par un ou plusieurs substituants choisis parmi G³ et/ou Z³ ;
G³ représente, à chaque fois qu'il est mentionné ci-dessus, un halogéno, un cyano, -N₃, -NO₂, -ONO₂ ou -A¹¹-R¹³ ;
où A¹¹ représente une liaison simple ou un groupe espaceur choisi parmi -C(O)A¹²-, -S(O)ₙA¹³-, -N(R¹⁴)A¹⁴-ou -OA¹⁵-, dans lesquels :
A¹² et A¹³ représentent indépendamment une liaison simple, -O-, -N(R¹⁴)- ou -C(O)- ;
A¹⁴ et A¹⁵ représentent indépendamment une liaison simple, -C(O)-, -C(O)N(R¹⁴)-, -C(O)O-, -S(O)ₙ- ou -S(O)ₙN(R¹⁴)- ;
Z³ représente, à chaque fois qu'il est mentionné ci-dessus, =O, =S, =NOR¹³, =NS(O)ₙN(R¹⁴) (R¹³), =NCN ou =C(H)NO₂ ;
n représente, à chaque fois qu'il est mentionné ci-dessus, 1 ou 2 ;
R¹³ et R¹⁴ sont indépendamment choisis parmi :
i) un hydrogène ;
ii) un alkyle en C₁₋₆ ou un groupe hétérocycloalkyle, ces deux groupes étant facultativement substitués par un ou plusieurs substituants choisis parmi un halogéno, un alkyle en C₁₋₄, -N(R¹⁵) (R¹⁶), -O(R¹⁵) et =O ; et
iii) un groupe aryle ou hétéroaryle, ces deux groupes étant facultativement substitués par un ou plusieurs substituants choisis parmi un halogéno, un alkyle en C₁₋₄, -N(R¹⁵) (R¹⁶) et -O(R¹⁵) ; ou
toute paire R¹³ et R¹⁴ peut, par exemple lorsqu'elle est présente sur des atomes identiques ou adjacents, être liée ensemble pour former avec ces atomes, ou d'autres appropriés, un autre cycle à 3 à 8 chaînons, contenant facultativement 1 à 3 hétéroatomes et/ou 1 à 3 liaisons doubles, lequel cycle est facultativement substitué par un ou plusieurs substituants choisis parmi un halogéno, un alkyle en C₁₋₄, -N(R¹⁵) (R¹⁶), -O(R¹⁵) et =O ;
R¹⁵ et R¹⁶ sont indépendamment choisis parmi un hydrogène et un alkyle en C₁₋₄, ce dernier groupe étant facultativement substitué par un ou plusieurs groupes halogéno ;
ou un sel pharmaceutiquement acceptable de celui-ci.

2. Composé selon la revendication 1, dans lequel :
A² et A³ représentent indépendamment une liaison simple, -O- ou -N(R¹⁰)- ;
Z¹ représente, à chaque fois qu'il est mentionné ci-dessus, =O, =NOR⁹, =NS(O)ₙN(R¹⁰) (R⁹), =NCN ou =C(H)NO₂ ;
A⁷ et A⁸ représentent indépendamment une liaison simple, -O- ou -N(R¹²) - ;
Z² représente, à chaque fois qu'il est mentionné ci-dessus, =O, =NOR¹¹, =NS(O)ₙN(R¹²)(R¹¹), =NCN ou =C(H)NO₂ ;
A¹² et A¹³ représentent indépendamment une liaison simple, -O- ou -N(R¹⁴)- ; et/ou
Z³ représente, à chaque fois qu'il est mentionné ci-dessus, =O, =NOR¹³, =NS(O)ₙ(R¹⁴)(R¹³), =NCN ou =C(H)NO₂.

3. Composé selon la revendication 1 ou la revendication 2, dans lequel n représente 2.

4. Composé selon l'une quelconque des revendications précédentes, dans lequel X représente une liaison simple ou -C(O)-.

5. Composé selon l'une quelconque des revendications précédentes, dans lequel A représente G¹.

6. Composé selon l'une quelconque des revendications précédentes, dans lequel G¹ représente un halogéno ou -A¹-R⁹.

7. Composé selon l'une quelconque des revendications précédentes, dans lequel A¹ représente une liaison simple, -OA⁵- ou -N(R¹⁰)A⁴-.

8. Composé selon l'une quelconque des revendications précédentes, dans lequel A⁴ et A⁵ représentent indépendamment une liaison simple.

9. Composé selon l'une quelconque des revendications précédentes, dans lequel B représente G².

10. Composé selon l'une quelconque des revendications précédentes, dans lequel G² représente un halogéno ou -OR¹¹.

11. Composé selon l'une quelconque des revendications précédentes, dans lequel R⁹ représente un alkyle en C₁₋₆, lequel groupe est facultativement substitué par un ou plusieurs groupes halogéno, ou un groupe phényle ou pyridyle.

12. Composé selon l'une quelconque des revendications précédentes, dans lequel R¹⁰ représente un alkyle en C₁₋₂.

13. Composé selon l'une quelconque des revendications précédentes, dans lequel R¹¹ représente un alkyle en C₁₋₂ facultativement substitué par un ou plusieurs groupes halogéno.

14. Composé selon l'une quelconque des revendications précédentes, dans lequel R¹ représente un groupe phényle ou pyridyle facultativement substitué.

15. Composé selon l'une quelconque des revendications précédentes, dans lequel R³ et R⁴ représentent indépendamment G¹, un hydrogène ou un groupe phényle ou pyridyle facultativement substitué.

16. Composé selon la revendication 15, dans lequel R³ et R⁴ représentent indépendamment un hydrogène ou un groupe phényle ou pyridyle facultativement substitué.

17. Composé selon l'une quelconque des revendications précédentes, dans lequel au moins l'un parmi R³ et R⁴ représente un phényle ou pyridyle facultativement substitué, et au plus un autre représente G¹ ou un hydrogène.

18. Composé selon l'une quelconque des revendications 15 à 17, dans lequel, lorsque R³ ou R⁴ représente un groupe phényle ou pyridyle facultativement substitué, alors les autres substituants sur le cycle benzénique essentiel de l'indole de formule I, tel que défini dans la revendication 1 (à savoir, R², R⁵ et R³ ou R⁴ (comme il convient)), représentent un hydrogène ou G¹.

19. Composé selon l'une quelconque des revendications précédentes, dans lequel R⁶ représente H
ou un groupe alkyle en C₁₋₄ facultativement substitué.

20. Composé selon l'une quelconque des revendications précédentes, dans lequel R⁷ représente un groupe alkyle en C₁₋₄ facultativement substitué ou un groupe phényle ou pyridyle facultativement substitué.

21. Composé selon l'une quelconque des revendications 1 à 18, dans lequel R⁶ et R⁷ sont liés pour former, conjointement avec l'atome d'azote et le groupe X auxquels ils sont respectivement attachés, un cycle à 5 à 6 chaînons, contenant facultativement 1 à 2 hétéroatomes.

22. Composé selon l'une quelconque des revendications 14 à 20, dans lequel les substituants facultatifs sont choisis parmi un cyano, un halogéno, un alkyle en C₁₋₆, lequel groupe alkyle peut être linéaire ou ramifié, cyclique, partiellement cyclique, insaturé et/ou facultativement substitué par un ou plusieurs groupes halogéno, et -OR¹⁷, où R¹⁷ représente H ou un alkyle en C₁₋₆ (lequel groupe alkyle est facultativement substitué par un ou plusieurs groupes halogéno).

23. Composé selon la revendication 22, dans lequel les substituants facultatifs sont choisis parmi un halogéno, un alkyle en C₁₋₆, lequel groupe alkyle peut être linéaire ou ramifié, cyclique, partiellement cyclique, insaturé et/ou facultativement substitué par un ou plusieurs groupes halogéno, et -OR¹⁷, où R¹⁷ représente H ou un alkyle en C₁₋₆ (lequel groupe alkyle est facultativement substitué par un ou plusieurs groupes halogéno).

24. Composé selon l'une quelconque des revendications précédentes, dans lequel R⁸ représente un hydrogène.

25. Composé selon l'une quelconque des revendications 1 à 24, ou sel pharmaceutiquement acceptable de celui-ci, pour l'utilisation en tant que produit pharmaceutique.

26. Formulation pharmaceutique incluant un composé tel que défini dans l'une quelconque des revendications 1 à 24 ou un sel pharmaceutiquement acceptable de celui-ci, en mélange avec un adjuvant, un diluant ou un véhicule pharmaceutiquement acceptable.

27. Utilisation d'un composé tel que défini dans l'une quelconque des revendications 1 à 24, ou d'un sel pharmaceutiquement acceptable de celui-ci, pour la fabrication d'un médicament pour le traitement d'une maladie dans laquelle une inhibition de l'activité de la prostaglandine E synthase-1 microsomale, de la leucotriène C₄ synthase et/ou de la protéine activant la 5-lipoxygénase est souhaitée et/ou requise.

28. Utilisation selon la revendication 27, dans laquelle l'inhibition de l'activité de la prostaglandine E synthase-1 microsomale est souhaitée et/ou requise.

29. Utilisation selon la revendication 27 ou la revendication 28, dans laquelle la maladie est une inflammation.

30. Utilisation selon la revendication 29, dans laquelle la maladie est une maladie intestinale inflammatoire, un syndrome du côlon irritable, une migraine, une céphalée, une douleur lombaire, une fibromyalgie, un trouble myofascial, une infection virale, une infection bactérienne, une infection fongique, une dysménorrhée, une brûlure, une opération chirurgicale ou dentaire, une malignité, une athérosclérose, une goutte, une arthrite, une arthrose, une arthrite juvénile, une polyarthrite rhumatoïde, une fièvre, une spondylarthrite ankylosante, un lupus érythémateux systémique, une vascularite, une pancréatite, une néphrite, une bursite, une conjonctivite, une iritis, une sclérite, une uvéite, une cicatrisation de blessure, une dermatite, un eczéma, un psoriasis, une attaque, un diabète, un trouble neurodégénératif, une maladie auto-immune, une ostéoporose, un asthme, une maladie pulmonaire chronique obstructive, une fibrose pulmonaire, un trouble allergique, une rhinite, un ulcère, une maladie coronarienne, une sarcoïdose, une douleur inflammatoire, un syndrome d'hyperprostaglandine E, un syndrome de Bartter classique, une maladie de Hodgkin, un canal persistant, toute autre maladie ayant une composante inflammatoire, une maladie de Paget ou une maladie parodontale.

31. Produit de combinaison, comprenant :
(A) un composé tel que défini dans l'une quelconque des revendications 1 à 24, ou un sel pharmaceutiquement acceptable de celui-ci ; et
(B) un autre agent thérapeutique qui est utile dans le traitement d'une inflammation, dans lequel chacun des constituants (A) et (B) est formulé en mélange avec un adjuvant, diluant ou véhicule pharmaceutiquement acceptable.

32. Produit de combinaison selon la revendication 31, qui comprend une formulation pharmaceutique incluant un composé tel que défini dans l'une quelconque des revendications 1 à 24, ou un sel pharmaceutiquement acceptable de celui-ci, un autre agent thérapeutique qui est utile dans le traitement d'une inflammation, et un adjuvant, diluant ou véhicule pharmaceutiquement acceptable.

33. Produit de combinaison selon la revendication 31, qui comprend un kit de pièces comprenant des constituants :
(a) une formulation pharmaceutique incluant un composé tel que défini dans l'une quelconque des revendications 1 à 24, ou un sel pharmaceutiquement acceptable de celui-ci, en mélange avec un adjuvant, diluant ou véhicule pharmaceutiquement acceptable ; et
(b) une formulation pharmaceutique incluant un autre agent thérapeutique qui est utile dans le traitement d'une inflammation, en mélange avec un adjuvant, diluant ou véhicule pharmaceutiquement acceptable,
lesquels constituants (a) et (b) sont chacun fournis sous une forme qui convient à l'administration conjointe avec l'autre.

34. Procédé pour la préparation d'un composé tel que défini dans la revendication 1, qui comprend :
(i) la réaction d'un composé de formule II, 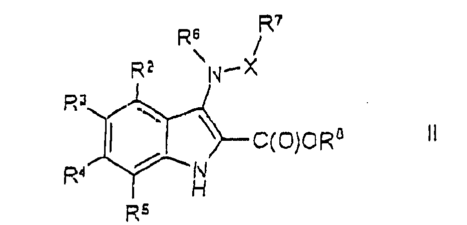 dans laquelle X, R², R³, R⁴, R⁵, R⁶, R⁷ et R⁸ sont tels que définis dans la revendication 1, avec un composé de formule III,
R¹L¹ III
dans laquelle L¹ représente un groupe partant approprié et R¹ est tel que défini dans la revendication 1 ;
(ii) la réaction d'un composé de formule IV, 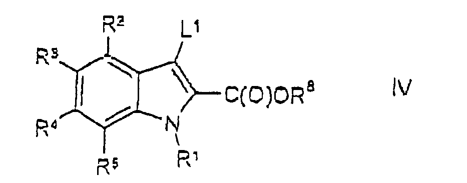 dans laquelle L¹ est tel que défini ci-dessus et R¹, R², R³, R⁴, R⁵ et R⁶ sont tels que définis dans la revendication 1, avec un composé de formule V,
HN(R⁶)XR⁷ V
dans laquelle X, R⁶ et R⁷ sont tels que définis dans la revendication 1 ;
(iii) la réaction d'un composé de formule VI, 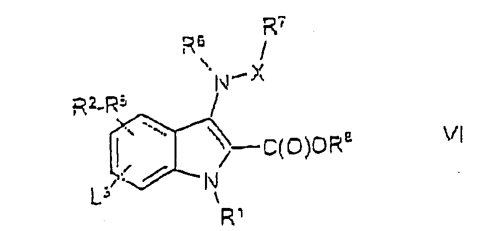 dans laquelle L³ représente L¹ ou L², où L² représente un groupe partant approprié et est attaché à un ou plusieurs des atomes de carbone du cycle benzénoïdique de l'indole, et dans laquelle les positions restantes du cycle benzénoïdique sont substituées par 1 à 3 (en fonction du nombre de substituants L³) substituants R² à R⁵, R² à R⁵ représentent l'un quelconque des sustituants, à savoir R ² R³, R⁴ et R⁵, qui sont déjà présents dans ce cycle (comme il convient), L¹ est tel que défini ci-dessus et X, R¹, R², R³, R⁴, R⁵, R⁶, R⁷ et R⁸ sont tels que définis dans la revendication 1, avec un composé de formule VII,
R¹⁸L⁴ VII
dans laquelle R¹⁸ représente R², R³, R⁴ ou R⁵ (comme il convient) et L⁴ représente L¹ (lorsque L³ est L²) ou L² (lorsque L³ est L¹) tels que définis ci-dessus ;
(iv) la réaction d'un composé de formule VIII, 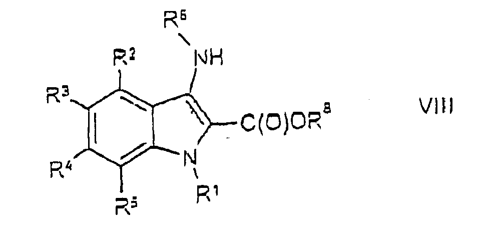 dans laquelle R¹, R², R³, R⁴, R⁵, R⁶ et R⁸ sont tels que définis dans la revendication 1, avec un composé de formule IX,
R⁷XL¹ IX
dans laquelle L¹ est tel que défini ci-dessus et X et R⁷ sont tels que définis dans la revendication 1 ; ou
(v) pour les composés de formule I dans lesquels X représente une liaison simple et R⁷ est un groupe alkyle en C₁₋₈, la réduction d'un composé de formule 1, dans lequel X représente -C(O)- et R⁷ représente H ou un groupe alkyle en C₁₋₇.
